# FASCICULE DE BREVET EUROPEEN

(11) **EP 0 367 644 B1**
(45) Date de publication et mention de la délivrance du brevet: **22.02.1995**
(21) Numéro de dépôt: 89402764.8
(22) Date de dépôt: 06.10.1989
(51) Int. Cl.: C12N 15/55, C12N 9/78, C12Q 1/68, G01N 33/569, A61K 39/106, C12P 21/02

(54) **Séquence de nucléotides codant pour une proteine à activité uréasique**
Nukleotidesequenzen, die ein Protein mit Ureareaktivität kodieren
Nucleotide sequence codings for a protein with urea reactivity

(30) Priorité: 06.10.1988 FR 8813135
(43) Date de publication de la demande: 09.05.1990
(60) Demande divisionnaire: 94110776.5
(73) Titulaire: INSTITUT PASTEUR, 75724 Paris Cédex 15 (FR); INSTITUT NATIONAL DE LA SANTE ET DE LA RECHERCHE MEDICALE (INSERM), 75654 Paris Cédex 13 (FR)
(72) Inventeur: Labigne, Agnès, F-91440 Bures sur Yvette (FR)
(74) Mandataire: Gutmann, Ernest

(56) Documents cités:
- EP-A- 0 200 362
- EP-A- 0 204 438
- WO-A-86/04422
- WO-A-87/01119
- CHEMICAL ABSTRACTS, vol. 109, no. 7, 15 Aout 88, p. 289 Columbus, Ohio, USA H.L.T. MOBLEY et al. & J. Clin. Microbiol. 1988, vol. 26, no. 5, pp. 831-836
- idem
- JOURNAL OF BACTERIOLOGY, vol. 169, no. 11, Novembre 1987 pp. 5320-5323, Baltimore, USA A. LABIGNE-ROUSSEL et al.
- CHEMICAL ABSTRACTS, vol. 108, no. 19, 9 Mai 1988, abstract no. 164225b, Columbus, Ohio, USA; S. MAJEWSKI et al. & J. Infect. Dis. 1988, vol. 157, no. 3, pp. 465-471
- JOURNAL OF MEDICAL MICROBIOLOGY, vol. 27, no. 1, 1988, pp. 38-40, Edinburgh, GB; R.L. FERRERO et al.
- CHEMICAL ABSTRACTS, vol. 110, no. 13, 27 mars 1989, p. 199 abrege, no. 109392v, Columbus, Ohio, USA; C.L. CLAYTON et al. & Infect. Immun. 1989, vol. 57, no. 2, pp. 623-629

## Description

L'invention a pour objet des séquences de nucléotides codant pour une uréase telle qu'exprimée naturellement chez Campylobacter pylori et ses applications biologiques, notamment pour la détection de C.pylori chez l'homme ou dans l'environnement.

C.pylori est une bactérie à gras négatif retrouvée à ce jour exclusivement à la surface de la muqueuse de la partie antrale de l'estomac chez l'homme, et plus particulièrement autour des lésions et des cratères des ulcères gastriques et duodénaux. 25% de la population est porteuse de C.pylori : 8% présentent une maladie ulcéreuse, 9% souffrent de dyspepsie non-ulcéreuse et 8% sont porteurs asymptomatiques.

Tous les C.pylori isolés et décrits à ce jour, possèdent les trois propriétés suivantes :
Les bactéries produisent une uréase dotée d'une grande activité.

Elles adhèrent très fortement aux cellules épithéliales de la muqueuse gastrique, cette propriété se traduisant in vitro par une très forte adhésion aux cellules Hela.

Les C.pylori produisent et libèrent une enzyme présentant une activité protéolytique conduisant à la dégradation du mucus et donc à un affaiblissement de la barrière naturelle protégeant la muqueuse gastrique.

La détection de C.pylori in situ, chez l'homme et dans l'environnement et l'étude de son pouvoir pathogène - elle est considérée comme responsable des gastrites actives chez l'homme - se heurtent toutefois aux difficultés de culture de cet organisme.

Sa croissance est très lente (6 à 7 jours sur milieu gélosé au sang), et doit s'effectuer dans des conditions de microaérophilie, l'oxygène de l'air étant toxique.

La recherche de moyens de détection de cette bactérie a conduit les inventeurs à identifier les gènes responsables chez C.pylori de la production d'uréase.

La détermination de la séquence de nucléotides d'un fragment intragénique a permis de disposer d'un outil utilisable comme sonde de détection pour l'identification spécifique des C.pylori.

L'invention a donc pour but de fournir de nouvelles séquences de nucléotides capables de coder pour des protéines à activité uréasique telles qu'exprimées chez C.pylori.

Elle a également pour but de fournir des fragments de cette séquence utilisables comme sondes de détection de C.pylori.

L'invention vise, en outre, à fournir une protéine à activité uréasique telle qu'exprimée chez C.pylori, de pureté élevée, et des fragments de cette protéine.

La séquence de nucléotides selon l'invention est caractérisée en ce qu'elle comprend au moins une partie d'une séquence codant pour une protéine à activité uréase telle qu'exprimée chez C.pylori.

L'invention a plus particulièrement pour objet une séquence de nucléotides capable de s'hybrider avec des gènes codant pour une protéine à activité uréase telle qu'exprimée chez C.pylori, dans les conditions suivantes : 68°C, 6 x SSC (1 x SSC est constitué de 0,15M de NaCl et 0,015M de citrate de sodium, pH7), en milieu Denhardt (1% Ficoll, 1% polyvinylpyrrolidone, 1% de sérum albumine de boeuf).

Selon un autre aspect de l'invention, la séquence de nucléotides est caractérisée en ce qu'elle porte l'information requise pour la production d'une protéine à activité uréase ou de ses fragments, capables de former un complexe immunologique avec des anticorps dirigés respectivement contre une protéine d'activité uréase telle qu'exprimée chez C.pylori ou contre des fragments de cette dernière.

La séquence selon l'invention est également caractérisée en ce qu'elle comprend au moins une partie d'un fragment d'environ 8kb correspondant au fragment de restriction EcoRI (ClaI, BamHI)-PstI (Hind III).

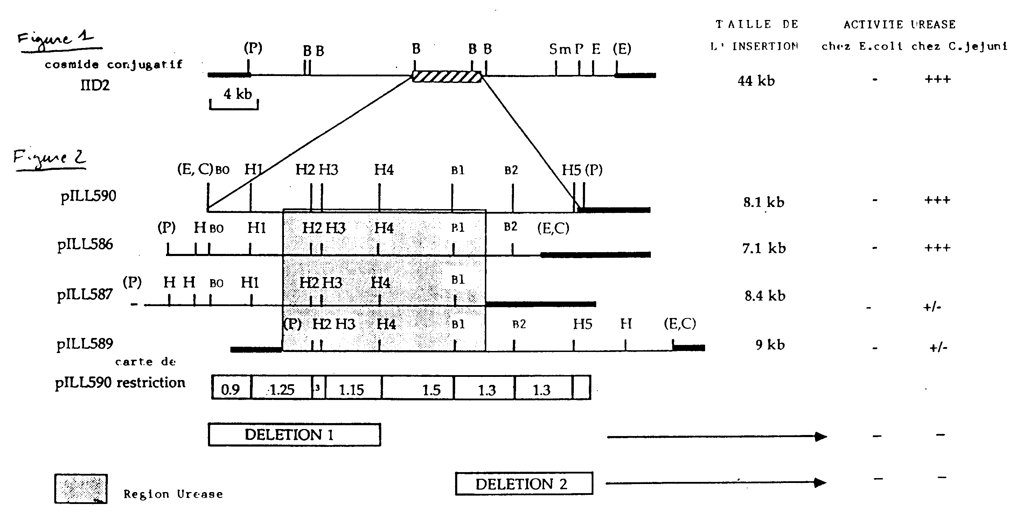

La carte de restriction enzymatique fragment est représentée sur la figure 2.

Une séquence plus spécialement préférée comprend au moins une partie d'un fragment d'environ 4,2kb (±5%) délimité par les nucléotides d'extrémité situés respectivement à une distance d'environ 0,55kb en amont du site H2 représenté sur la figure 2, et d'environ 0,7 kb en aval du site B1 représenté sur la figure 2.

Lés séquences constituées par ce fragment portent l'information nécessaire pour l'expression d'une protéine à activité uréase telle qu'exprimée naturellement chez C.pylori.

L'expression de ces séquences peut intervenir chez tout autre microorganisme capable d'exploiter à son profit l'information portée par ces séquences, notamment chez une souche de Campylobacter naturellement déficiente en gènes codant pour la production d'une protéine à activité uréase (ou encore souche de campylobacter uréase⁻).

Selon encore un autre aspect, l'invention vise une séquence recombinante comprenant l'une des séquences définies ci-dessus, le cas échéant associée à un promoteur capable de contrôler la transcription de la séquence et une séquence d'ADN codant pour des signaux de terminaison de la transcription.

Les séquences de nucléotides de l'invention sont caractérisées en ce qu'elles sont capables de s'hybrider avec une sonde formée à partir de la séquence présentant:
- l'enchaînement (I) de nucléotides suivant: 
AAGCTTTTAGGGGTGTTAGGGGTTTATCAAAAATCTAAAAACGCCCTTTCTTCTCAAGCA
ATTGTCGCTACAAACATGAGCAATTTAGCCCTTAAAGAATACTTAAAATCCCAAGATTTA
GAATTGAAGCATTGCGCGATTGGGGATAAGTTTGTGAGCGAATGCATGCGATTGAACAAA
GCCAATTTTGGAGGCGAGCAAAGCGGCATATCATTTTTAGCGATTACGCTAAAACCGGC
GATGGCTTGGTGTGCGCTTTGCAAGTGAGCGCGTTAGTGTTAGAAAGTAAGCTT
- ou l'enchaînement (II) de nucléotides suivant: 
AACGCATGATTGATTGCAGAAGGAGTGGTGCCCCAGTCTTCGTGAATTTTAAAGCCAATC
GCACCGGCTTCAATTTGATCGGCTAAGCTCGCATCGTTAGAAGCGTTACCTTTAGCCAAG
AAACCTAAATTCATAGAATATTCTTCAGCCGCTCTGAGCATCCATTTTAAATTTCTTCTG
CCTGGAGTGATAGTAGTCGCATTAGTGCCATCAGCAGGACCGGTTCCACCACCAATCATG
GTTGTTACACCGCTTCGAAAAGCTGTAGGGATTTGTTGCGGTGAAATGAAGTGGATGTGT
GTGTCAATACCACCAGCCGTTACGATCAAACCTTCACCGGCTAAGGCTTCAGTAGCAGGA
CCTACGCTAAGATTGTTTTTAACGCCATCTTGCATGTCTTTGTTACCGCCTTTACCAATG
CCAGCGATTTTGCCATCTTTAATACCAATATCCGCTTTATAAATACCGGTGTAATCCACG
ATTAAAGCGTTAGTGATAATTAAATCCAACTCTTCTTTGCTAGGGTTGTTAGATTGGCTC
ATGCCTTCTCTTAGGGGTTTTACCGCCACCGAATTTAAGCTCTTCGCCATAAATGGTGTA
GTCATGTTCTACTTCAGCGATCAAGTCTGATCGCCCAATCTCACTTTATCGCCTGTAGTA
GGACCATACATAGAAACATATTCTTTTCTGCTAATCTTTTTCATTTCTTACTCCTTAATT
GTTTTTACATAGTTGTCATCGCTTTTAGG
 ou l'enchaînement (IIbis) de nucléotides suivant: 
CCTAAAAGCGATGACAACTATGTAAAAACAATTAAGGAGTAAGAAATGAAAAAGATTAGC
AGAAAAGAATATGTTTCTATGTATGGTCCTACTACAGGCGATAAAGTGAGATTGGCGAT
CAGACTTGATCGCTGAAGTAGAACATGACTACACCATTTATGGCGAAGAGCTTAAATTCG
GTGGCGGTAAAACCCCTAAGAGAAGGCATGAGCCAATCTAACAACCCTAGCAAAGAAGAG
TTGGATTTAATTATCACTAACGCTTTAATCGTGGATTACACCGGTATTTATAAAGCGGAT
ATTGGTATTAAAGATGGCAAAATCGCTGGCATTGGTAAAGGCGGTAACAAAGACATGCAA
GATGGCGTTAAAAACAATCTTAGCGTAGGTCCTGCTACTGAAGCCTTAGCCGGTGAAGGT
TTGATCGTAACGGCTGGTGGTATTGACACACACATCCACTTCATTTCACCCCAACAAATC
CCTACAGCTTTTCGAAGCGGTGTAACAACCATGATTGGTGGTGGAACCGGTCCTGCTGAT
GGCACTAATGCGACTACTATCACTCCAGGCAGAAGAAATTTAAAATGGATGCTCAGAGCG
GCTGAAGAATATTCTATGAATTTAGGTTTCTTGGCTAAAGGTAACGCTTCTAACGATGCG
AGCTTAGCCGATCAAATTGAAGCCGGTGCGATTGGCTTTAAAATTCACGAAGACTGGGGC
ACCACTCCTTCTGCAATCAATCATGCGTT
- ou les enchaînements de nucléotides complémentaires respectivement des enchaînements (I) (II) et (IIbis) susmentionnés.

Des séquences de nucléotides selon l'invention, sont caractérisées en ce qu'elles comprennent les enchaînements de nucléotides définis ci-dessus ou sont constituées au moins par une partie de ces enchaînements.

Il va de soi que les bases des séquences de nucléotides considérées peuvent être dans un ordre différent de celui trouvé dans les gènes et/ou que ces bases peuvent être, le cas échéant, substituées, dès lors qu'une sonde élaborée à partir de telles séquences donne une réponse caractéristique et non équivoque quant à la capacité de reconnaître la présence de gènes codant pour la protéine à activité uréase de C.pylori.

Toute séquence de nucléotides hybridable avec celle des enchaînements de nucléotides sus-mentionnés telle qu'obtenue par transcription enzymatique inverse de l'ARN correspondant ou encore par synthèse chimique, entre également dans le cadre de l'invention.

L'invention concerne également une séquence de nucléotides correspondant, selon le code génétique universel, à au moins une partie de la séquence (III) en acides aminés suivante : 
KLLGVLGVYQKSKNALSSQAIVATNMSNLALKEYLKSQDLELKHCAIGDKFVSECMRLNK
ANFGGEQSGHIIFSDYAKTGDGLVCALQVSALVLESKL
L'invention a également pour objet une séquence de nucléotides correspondant, selon le code génétique universel, à au moins une partie de la séquence en acides aminés (IV) suivante (codée par la séquence nucléotidique (IIbis) 
NMTTPFMAKSLNSVAVKPLREGMSQSNNPSKEELDLIITNALIVDYTGIYKADIGIKDGK
IAGIGKGGNKDMQDGVKNNLSVGPATEALAGEGLIVTAGGIDTHIHFISPQQIPTAFRSG
VTTMIGGGTGPADGTNATTITPGRRNLKWMLRAAEEYSMNLGFLAKGNASNDASLADQIE
AGAIGFKIHEDWGTTPSAINHA
L'invention se rapporte également aux vecteurs recombinants d'expression et de clonage, capables de transformer une cellule hôte appropriée, comportant au moins une partie d'une séquence de nucléotides telle que définie ci-dessus sous le contrôle d'éléments de régulation permettant son expression.

Des vecteurs recombinants préférés renferment au moins une partie de la séquence d'environ 4,2kb évoquée plus haut.

Les souches de microorganismes transformées entrent également dans le cadre de l'invention. Ces souches comportent l'une des séquences de nucléotides définies ci-dessus ou encore un vecteur recombinant tel que défini précédemment.

La souche E.coli S17-1 déposée le 16 Août 1988 sous le n° I-795 à la Collection Nationale de Culture de Microorganismes (C.N.C.M.) de l'Institut Pasteur à PARIS (FRANCE) porte le plasmide pILL590 de 16,3kb environ qui comprend le fragment de restriction d'environ 8kb EcoRI(ClaI, BamHI)-PstI (Hind III) évoqué plus haut.

L'invention vise en outre une protéine présentant une activité uréase du type de celle exprimée naturellement chez C.pylori ainsi que les fragments peptidiques de cette protéine.

La protéine de l'invention, et ses fragments, correspondent selon le code génétique universel, aux séquences de nucléotides définies ci-dessus, en particulier à au moins une partie de la séquence d'environ 4,2 kb.

La protéine de l'invention est également caractérisée en ce qu'il s'agit d'une protéine telle qu'obtenue par transformation de cellules hôtes au moyen d'un vecteur recombinant comme défini ci-dessus, mise en culture, dans un milieu approprié, des cellules hôtes transformées et récupération de la protéine à partir de ces cellules ou directement à partir du milieu de culture. La production d'uréase ou de fragments de cette dernière par ce procédé, fait également partie de l'invention.

La protéine de l'invention et ses fragments, qui peuvent être également obtenus par synthèse chimique, présentent avantageusement un degré de pureté élevé et sont utilisés pour former, selon les techniques classiques, des anticorps polyclonaux.

De tels anticorps polyclonaux, ainsi que les anticorps monoclonaux capables de reconnaître spécifiquement la protéine ci-dessus et ses fragments sont également visés par l'invention.

Les séquences de nucléotides définies ci-dessus sont obtenues selon les techniques classiques du génie génétique par clonage et identification des gènes responsables de la synthèse d'une protéine à activité uréase chez C.pylori.

L'invention vise également les applications biologiques des séquences de nucléotides, des protéines correspondantes et des anticorps monoclonaux ou polyclonaux.

Ces applications comprennent l'élaboration, à partir de fragments intragéniques, de sondes pour la détection de C.pylori. Cette élaboration comprend, notamment, la dénaturation des séquences double-brin pour obtenir une séquence monobrin utilisable en tant que sonde.

Les essais réalisés avec de tels fragments pour détecter la présence éventuelle de séquences complémentaires chez divers Campylobacter et chez des microorganismes appartenant à des genres différents ont mis en évidence la grande spécificité de ces fragments.

L'invention vise donc des sondes de détection caractérisées en ce qu'elles comprennent au moins une partie d'une séquence de nucléotides définie ci-dessus.

Toute sonde ne se distinguant de la précédente, au niveau de sa séquence de nucléotides, que par des substitutions ou altérations de nucléotides n'entraînant pas de modification de ses propriétés d'hybridation avec le génome de C.pylori, entre dans le cadre de l'invention.

Le fragment d'ADN utilisé comme sonde comporte un nombre de nucléotides suffisant pour obtenir la spécificité requise et la formation d'un hybride stable.

Il est possible d'utiliser des fragments atteignant plusieurs kb, des résultats de haute spécificité étant cependant également obtenus avec des fragments plus courts d'environ 25 à 40 nucléotides.

Des sondes appropriées pour ce type de détection sont avantageusement marquées par un élément radio-actif ou tout autre groupe permettant sa reconnaissance à l'état hybridé avec la préparation renfermant l'ADN à étudier.

Selon les techniques classiques, ces sondes sont mises en contact avec un échantillon biologique renfermant des bactéries, ou directement avec ces bactéries ou leurs acides nucléiques, dans des conditions autorisant l'hybridation éventuelle de la séquence de nucléotides de la sonde avec une séquence complémentaire, éventuellement contenue dans le produit testé.

On peut, par exemple, mettre en oeuvre la méthode d'hybridation sur taches. Cette méthode comporte après dénaturation de l'ADN préalablement obtenu à partir de bactéries provenant de biopsies antrales, le dépôt d'une quantité aliquote de cet ADN sur des membranes de nitrocellulose, l'hybridation de chaque membrane dans les conditions usuelles avec la sonde et la détection, de manière classique, de l'hybride formé.

On peut aussi utiliser une méthode d'hybridation sur réplique, selon la technique de Southern. Cette méthode comprend la séparation électrophorétique en gel d'agarose des fragments d'ADNs engendrés après traitement de l'ADN par des enzymes de restriction, le transfert après dénaturation alcaline sur des membranes appropriées et leur hybridation avec la sonde dans les conditions usuelles. Il n'est pas toujours nécessaire de procéder à l'expression préalable de l'ADN. Il suffit que l'ADN soit rendu accessible à la sonde.

La détection pour l'identification spécifique des C. pylori peut être également réalisée par des techniques d'amplification de l'ADN (PCR). Ces techniques sont décrites en particulier dans les brevets US 4683202 et 4683195 au nom de Cetus Corporation.

Pour la mise en oeuvre de ces techniques, on utilise deux amorces de 10 à 40 nucléotides, avantageusement d'environ une vingtaine de nucléotides, ces amorces étant comprises dans l'une des séquences de nucléotides définies ci-dessus ou susceptibles de s'hybrider avec une partie d'une des séquences nucléotidiques défines ci-dessus, ou de leurs séquences complémentaires. De manière avantageuse, ces amorces sont distantes d'environ 200 à 250 nucléotides lorsqu'elles sont hybridées (ou liées), aux brins de l'ADN à amplifier. L'une des séquences est capable de se lier à une séquence de nucléotides d'un des brins de fragments d'ADN à amplifier, cette séquence étant située au niveau d'une extrémité de ce fragment (par exemple l'extrémité 5') ; l'autre séquence est capable de se lier à une séquence de nucléotides du deuxième brin du fragment d'ADN à amplifier, cette dernière séquence étant située au niveau de l'extrémité de ce fragment opposée à celle sus-mentionnée (cette dernière extrémité étant encore désignée par extrémité 3' dans l'exemple proposé).

Des amorces préférées sont comprises dans la séquence de nucléotides du fragment de restriction H2-H3 de la figure 2, et sont distantes d'environ 200 à 250 nucléotides.

Il s'agit, en particulier, des fragments respectivement à chaque extrémité de cette séquence de restriction HindIII-HindIII.

Un procédé de dépistage in vitro de la présence éventuelle de C. pylori dans un échantillon biologique comprend les étapes suivantes :
- éventuellement l'amplification préalable de la quantité de séquences de nucléotides susceptibles d'être contenues dans l'échantillon, par mise en contact de cet échantillon avec des amorces telles que décrites ci-dessus, ces amorces étant susceptibles respectivement de se lier d'une part à l'extrémité 5' d'un brin de ladite séquence de nculéotides et d'autre part, à l'exptrémité 3' de l'autre brin de ladite séquence de nucléotide, cette mise en contact étant suivie d'une étape d'amplification génique en présence d'une ADN polymérase et des quatre nucléosides triphosphates dATP, dCTP, dGTP, dTTP, ces opérations d'hybridation des amorces et d'amplification génique étant répétées plusieurs fois,
- la mise en contact de l'échantillon biologique en question avec une sonde nucléotidique selon l'invention, dans des conditions permettant la production d'un complexe d'hybridation formé entre la sonde et la séquence de nucléotides,
- la détection du complexe d'hybridation.

L'invention fournit ainsi des outils permettant de détecter rapidement C. pylori, in situ, et dans l'environnement avec une grande spécificité, sans avoir à pratiquer de cultures.

De tels outils de détection sont particulièrement utiles pour étudier le réservoir naturel de ces bactéries, les méthodes de transmission, de circulation et de contamination. De plus, dans le diagnostic in vitro pratiqué sur les biopsies, l'utilisation de ces sondes permet un gain de temps considérable par rapport aux techniques actuelles qui nécessitent de plus une technologie ne pouvant être réalisée que dans des services spécialisés, à savoir, des techniques bactériologiques ou utilisant la microscopie électronique.

Des résultats reproductibles ont été obtenus en utilisant comme sonde intragénique le fragment d'environ 8kb défini ci-dessus.

Compte tenu de leur spécificité à l'égard de C.pylori, ces sondes constituent également un outil de grand intérêt.

Pour étudier le pouvoir pathogène de C.pylori de façon à prévenir l'infection, il est ainsi possible d'étudier le rôle joué par C.pylori dans le développement des maladies ulcéreuses et d'identifier les déterminants génétiques qui semblent impliqués dans le pouvoir pathogène de cet organisme en vue de créer in vitro des mutants isogéniques de C.pylori, c.a.d. des bactéries modifiées génétiquement dans chacun des déterminants qui semblent jouer un rôle dans la pathogénèse de l'infection, mutants qui pourront être testés dans des modèles animaux.

L'invention concerne également la détection du polymorphisme des gènes codant pour l'uréase chez C.pylori à l'aide des sondes nucléotidiques définies ci-dessus et d'enzymes de restriction appropriées. Les conditions dans lesquelles cette détection est réalisée sont plus particulièrement décrites dans l'article de Gusella J.F. dans J. of Clin. - investigations (1986), 77, 1723-1726.

L'invention vise également les applications immunologiques de la protéine codée, plus spécialement pour l'élaboration d'antisérum spécifique ainsi que d'anticorps polyclonaux et monoclonaux. Les anticorps polyclonaux sont formés selon les techniques classiques par injection de la protéiné à des animaux, récupération des antisérums, puis des anticorps à partir des antisérums par exemple par chromatographie d'affinité.

Les anticorps monoclonaux sont produits de manière habituelle en fusionnant des cellules myélomes avec des cellules de rate d'animaux préalablement immunisés à l'aide des protéines de l'invention.

L'invention vise, en outre, un procédé de dépistage in vitro de la présence éventuelle de C.pylori dans un échantillon biologique susceptible de le contenir. Ce procédé est caractérisé en ce qu'il comprend :
- la mise en contact de l'échantillon avec un anticorps selon l'invention, dans des conditions permettant la production d'un complexe immunologique formé entra tout ou partie de la protéine à activité uréase produite par C.pylori et cet anticorps, et
- la détection du complexe immunologique.

Pour la mise en oeuvre des méthodes de dépistage in vitro, considérées ci-dessus basées sur l'utilisation de sondes nucléotidiques, on a recours avantageusement à des nécessaires ou kits comprenant :
- le cas échéant, au moins un couple d'amorces selon l'invention,
- une quantité déterminée d'une sonde nucléotidique selon l'invention,
- avantageusement, un milieu approprié respectivement à la formation d'une réaction d'hybridation entre la séquence à détecter et la sonde.
- avantageusement des réactifs permettant la détection des complexes d'hybridation formés entre la séquence de nucléotides et la sonde lors de la réaction d'hybridation.

Pour la mise en oeuvre des méthodes de dépistage in vitro définies ci-dessus, basées sur l'utilisation d'anticorps on aura recours avantageusement à des nécessaires ou kits, comprenant :
- une quantité déterminée d'un anticorps selon l'invention,
- avantageusement un milieu approprié à la formation d'une réaction immunologique entre au moins une partie de la protéine à activité uréase produite par une souche de C.pylori et l'anticorps,
- avantageusement, des réactifs permettant la détection des complexes immunologiques formés entre au moins une partie de la protéine à activité uréase et l'anticorps lors de la réaction immunologique.

Une étude approfondie du fragment de 4,2kb défini ci-dessus a permis de situer les gènes de structure codant pour les sous-unités polypeptidiques majeures de l'uréase de C.pylori.

Cette étude a été réalisée en construisant des mutants dans les plasmides combinants introduits chez E.coli et C.jejuni incapables de synthétiser l'uréase, (Baskerville-A, Newell D. (1988), Gut.29, 465-472). Le système de transcription-traduction in vitro d'E.coli ainsi qu'un système d'expression en minicellules ont été utilisés afin d'identifier et de localiser, au sein du fragment de 4,2kb de C.pylori, l'ensemble des gènes codant pour les sous-unités de l'uréase: deux protéines majeures de 26kDa et 61kDa ont été identifiées. Parallèlement, après diverses délétions au sein du fragment de 4,2kb, un système de transfert par conjugaison d'une souche d'E.coli à C.jejuni (du type de celui explicité dans la description détaillée qui suit) a été utilisé pour identifier les régions indispensables à l'expression de l'activité uréasique et corréler la délétion d'une région avec la disparition d'un polypeptide et la perte de l'activité uréasique. Il a été ainsi déterminé que les gènes codant pour les deux polypeptides nécessaires à l'expression de l'activité uréasique se situent dans la région centrale de 3,35kb, en aval du site H3 de la figure 2, et à cheval sur B1. La transcription des gènes codant pour les polypeptides de 61kDa ou de 26kDa se fait dans le sens EcoRI-PstI. Le fragment de 3,35kb est nécessaire mais non suffisant à l'expression de l'activité uréasique chez C.jejuni. Une région adjacente de 0,85kb, située en amont et apparemment non associée à l'expression de polypeptides chez E.coli, est indispensable à l'expression de l'activité uréasique chez C.jejuni.

L'analyse des mutants de délétion par transcription-traduction in vitro a permis d'observer que les deux polypeptides de 26 et 61kDa sont codés par deux fragments d'ADN adjacents.

La présente invention a pour objet tout ou partie de la séquence nucléotidique (V) suivante : 
1
ATGAAACTCACCCCAAAAGAGTTAGATAAGTTGATGCTCCACTACGCTGGAGAATTGGCT
AAAAAACGCAAAGAAAAAGGCATTAAGCTTAACTATGTAGAAGCAGTAGCTTTGATTAGT
GCCCATATTATGGAAGAAGCGAGAGCTGGTAAAAAGACTGCGGCTGAATTGATGCAAGAA
GGGCGCACTCTTTTAAAACCAGATGATGTGATGGATGGCGTGGCAAGCATGATCCATGAA
GTGGGTATTGAAGCGATGTTTCCTGATGGCACTAAACTCGTAACCGTGCATACCCCTATT
GAGGCCAATGGTAAATTAGTTCCTGGTGAGTTGTTCTTAAAAAATGAAGACATCACTATC
AACGAAGGCAAAAAAGCCGTTAGCGTGAAAGTTAAAAATGTTGGCGACAGACCGGTTCAA
ATCGGCTCACACTTCCATTTCTTTGAAGTGAATAGATGCCTAGACTTTGACAGAGAAAAA
ACTTTCGGTAAACGCTTAGACATTGCGAGCGGGACAGCGGTAAGATTTGAGCCTGGCGAA
GAAAAATCCGTAGAATTGATTGACATTGGCGGTAACAGAAGAATCTTTGGATTTAACGCA
TTGGTTGATAGACAAGCAGACAACGAAAGCAAAAAAATTGCTTTACACAGAGCTAAAGAG
CGTGGTTTTCATGGCGCTAAAAGCGATGACAACTATGTAAAAACAATTAAGGAGTAA
717
L'invention a plus particulièrement pour objet toute séquence nucléotidique correspondant, selon le code génétique universel à au moins une partie de la séquence en acides animés (VI) de 26kDa (codée par la séquence nucléotidique (V)) suivante: 
1
MKLTPKELDKLMLHYAGELAKKRKEKGIKLNYVEAVALISAHIMEEARAGKKTAAELMQE
GRTLLKPDDVMDGVASMIHEVGIEAMFPDGTKLVTVHTPIEANGKLVPGELFLKNEDITI
NEGKKAVSVKVKNVGDRPVQIGSHFHFFEVNRCLDFDREKTFGKRLDIASGTAVRFEPGE
EKSVELIDIGGNRRIFGFNALVDRQADNESKKIALHRAKERGFHGAKSDDNYVKTIKE
239
239 acides aminés
Poids moléculaire : 26.522 daltons
L'invention a également pour objet toute ou partie de la séquence nucléotidique (VII) suivantes: 
1
ATGAAAAAGATTAGCAGAAAAGAATATGTTTCTATGTATGGTCCTACTACAGGCGATAAA
GTGAGATTGGCGGATACAGACTTGATCGCTGAAGTAGAACATGACTACACCATTTATGGC
GAAGAGCTTAAATTCGGTGGCGGTAAAACCCTAAGAGAAGGCATGAGCCAATCTAACAAC
CCTAGCAAAGAAGAGTTGGATTTAATTATCACTAACGCTTTAATCGTGGATTACACCGGT
ATTTATAAAGCGGATATTGGTATTAAAGATGGCAAAATCGCTGGCATTGGTAAAGGCGGT
AACAAAGACATGCAAGATGGCGTTAAAAACAATCTTAGCGTAGGTCCTGCTACTGAAGCC
TTAGCCGGTGAAGGTTTGATCGTAACGGCTGGTGGTATTGACACACACATCCACTTCATT
TCACCCCAACAAATCCCTACAGCTTTTGCAAGCGGTGTAACAACCATGATTGGTGGTGGA
ACCGGTCCTGCTGATGGCACTAATGCGACTACTATCACTCCAGGCAGAAGAAATTTAAAA
TGGATGCTCAGAGCGGCTGAAGAATATTCTATGAATTTAGGTTTCTTGGCTAAAGGTAAC
GCTTCTAACGATGCGAGCTTAGCCGATCAAATTGAAGCCGGTGCGATTGGCTTTAAAATT
CACGAAGACTGGGGCACCACTCCTTCTGCAATCAATCATGCGTTAGATGTTGCGGACAAA
TACGATGTGCAAGTCGCTATCCACACAGACACTTTGAATGAAGCCGGTTGTGTAGAAGAC
ACTATGGCTGCTATTGCTGGACGCACTATGCACACTTTCCACACTGAAGGCGCTGGCGGC
GGACACGCTCCTGATATTATTAAAGTAGCCGGTGAACACAACATTCTTCCCGCTTCCACT
AACCCCACCATCCCTTTCACCGTGAATACAGAAGCAGAGCACATGGACATGCTTATGGTG
TGCCACCACTTGGATAAAAGCATTAAAGAAGATGTTCAGTTCGCTGATTCAAGGATCCGC
CCTCAAACCATTGCGGCTGAAGACACTTTGCATGACATGGGGATTTTCTCAATCACCAGT
TCTGACTCTCAAGCGATGGGCCGTGTGGGTGAAGTTATCACTAGAACTTGGCAAACAGCT
GACAAAAACAAGAAAGAATTTGGCCGCTTGAAAGAAGAAAAAGGCGATAACGACAACTTC
AGGATCAAACGCTACTTGTCTAAATACACCATTAACCCAGCGATCGCTCATGGATTAGC
GAGTATGTAGGTTCAGTAGAAGTGGGCAAAGTGGCTGACTTGGTATTGTGGAGTCCAGCA
TTCTTTGGCGTGAAACCCAACATGATCATCAAAGGCGGATTCATTGCGTTAAGCCAAATG
GGCGATGCGAACGCTTCTATCCCTACCCCACAACCGGTTTATTACAGAGAAATGTTCGCT
CATCATGGTAAAGCTAAATACGATGCAAACATCACTTTTGTGTCTCAAGCGGCTTATGAC
AAAGGCATTAAAGAAGAATTAGGACTTGAAAGACAAGTGTTGCCGGTAAAAAATTGCAGA
AATATCACTAAAAAAGACATGCAATTCAACGACACTACTGCTCACATTGAAGTCAATCCT
GAAACTTACCATGTGTTCGTGGATGGCAAAGAAGTAACTTCTAAACCAGCCAATAAAGTG
AGCTTGGCGCAACTCTTTAGCATTTTCTAG
1710
L'invention a plus particulièrement pour objet toute séquence nucléotidique correspondant, selon le code génétique universel à au moins une partie de la séquence en acides aminés (VIII) de 61kDa (codée par la séquence nucléotidique (VII)) suivante: 
1
MKKISRKEYVSMYGPTTGDKVRLGDTDLIAEVEHDYTIYGEELKFGGGKTLREGM
SQSNNPSKEELDLIITNALIVDYTGIYKADIGIKDGKIAGIGKGGNKDMQDGVKNN
LSVGPATEALAGEGLIVTAGGIDTHIHFISPQQIPTAFASGVTTMIGGGTGPAD
GTNATTITPGRRNLKWMLRAAEEYSMNLGFLAKGNASNDASLADQIEAGAIGF
KIHEDWGTTPSAINHALDVADKYDVQVAIHTDTLNEAGCVEDTMAAIAGRTMH
TFHTEGAGGGHAPDIIKVAGEHNILPASTNPTIPFTVNTEAEHMDMLMVCHHLD
KSIKEDVQFADSRIRPQTIAAEDTLHDMGIFSITSSDSQAMGRVGEVITRTWQT
ADKNKKEFGRLKEEKGDNDNFRIKRYLSKYTINPAIAHGISEYVGSVEVGKVADL
VLWSPAFFGVKPNMIIKGGFIALSQMGDANASIPTPQPVYYREMFAHHGKAKYD
ANITFVSQAAYDKGIKEELGLERQVLPVKNCRNITKKDMQFNDTTAHIEVNPETY
HVFVDGKEVTSKPANKVSLAQLFSIF
569
569 Acides aminés
Poids moléculaire : 61 729 daltons
La comparaison de la séquence en acides aminés VIII avec celle de l'uréase d'haricot (Jack Bean) décrit notamment dans Eur. J. Biochem 175, 151-165 (1588), montre de façon inattendue un haut niveau d'homologies d'acides aminés. Cette conservation de structure a permis aux inventeurs de déduire le site actif de la sous-unité uréasique de 61kDa qui est compris, en totalité ou en partie, dans la séquence polypeptidique délimitée par les acides aminés situés aux positions 206 et 338 de la séquence VIII décrite ci-dessus.

L'invention vise également toute ou partie de la séquence nucléotidique IX constituée successivement de la séquence V et de la séquence VII.

L'invention se rapporte également à toute séquence nucléotidique correspondant, selon le code génétique universel à au moins une partie de la séquence en acides aminés (X) de 87kDa constituée successivement de la séquence VI et de la séquence VIII.

L'invention concerne également des sondes de détection de la présence éventuelle de C.pylori dans un échantillon biologique, caractérisée en ce qu'elles comprennent au moins une partie d'une séquence de nucléotides définies ci-dessus.

Il va de soi que les bases des séquences de nucléotides considérées peuvent être dans un ordre différent de celui trouvé dans les gènes et/ou que ces bases peuvent être, le cas échéant, substituées, dès lors qu'une sonde élaborée à partir de telles séquences donne une réponse caractéristique et non équivoque quant à la capacité de reconnaître la présence de gènes codant pour la protéine à activité uréase de C.pylori.

Toute séquence de nucléotides hybridable avec celle des enchaînements de nucléotides sus-mentionnés telle qu'obtenue par transcription enzymatique inverse de l'ARN correspondant ou encore par synthèse chimique, entre également dans le cadre de l'invention.

Toute sonde ne se distiguant de la précédente, au niveau de sa séquence de nucléotides, que par des substitutions ou altérations de nucléotides n'entraînant pas de modification de ses propriétés d'hybridation avec le génome de C.pylori, entre dans le cadre de l'invention.

Les sondes sus-mentionnées sont utilisables dans des procédés de diagnostic on vitro de la présence éventuelle de C.pylori dans un échantillon biologique tels que décrits ci-dessus.

L'invention a également pour objet des amorces nucléotidiques d'environ 10 à environ 40 nuclétides, ces amorces étant comprises dans l'une des séquences nucléotidiques v ou VII définies ci-dessus (ou dans l'une des séquences dérivées de ces séquences V et VII), ou étant susceptibles de s'hybrider avec une partie d'une des séquences nucléotidiques susmentionnées ou avec leurs séquences complémentaires (notamment dans les conditions d'hybridation définies ci-dessus).

L'invention concerne également l'utilisation de ces amorces dans les méthodes de diagnostic in vitro définies ci-dessus, pour l'amplification préalable de la quantité de nucléotides de C.pylori susceptibles d'être contenus dans l'échantillon biologique selon le procédé décrit ci-dessus.

L'invention se rapporte également aux vecteurs recombinants d'expression et de clonage, capables de transformer une cellule hôte appropriée comportant toute ou partie d'une séquence nucléotidique identique ou dérivée des séquences nucléotidiques V, VII et IX sus-mentionnées.

L'invention vise également un procédé de production de protéines correspondat à toute ou partie des séquences VI, VIII et X sus-mentionnées, ce procédé comprenant la transformation de cellules hôtes appropriées (notamment celles décrites ci-dessus) à l'aide des vecteurs sus-mentionnés et la récupération des protéines ainsi produites suivie, le cas échant, par une étape de purification de ces protéines.

Le procédé de production de protéines défini ci-dessus comprend le cas échéant une étape préalable d'amplification de gène codant pour la protéine dont la production est recherchée, cette étape étant réalisée à l'aide de couples d'amorces de l'invention selon le procédé décrit ci-dessus.

L'invention a également pour objet les polypeptides (ou protéines) codés par les séquences nucléotidiques décrites ci-dessus. En particulier l'invention vise la séquence en acides aminés X de 87k Da la séquence VI de 26kDa, et la séquence VIII de 61kDa décrites ci-dessus ainsi que leurs fragments.

L'invention concerne également les anticorps polyclonaux et monoclonaux dirigés contre toute ou partie des polypeptides décrits ci-dessus et susceptibles de former un complexe immunologique avec eux.

L'invention vise particulièrement les anticorps, notamment monoclonaux, obtenus à l'aide d'une séquence peptidique d'au moins environ 10 à 15 acides aminés de la séquence VI, ou de la séquence VIII.

Des anticorps monoclonaux préférés de l'invention sont dirigés contre toute ou partie de la séquence délimitée par les acides aminés situés aux positions 206 et 338 de la séquence VIII, et plus particulièrement ceux obtenus à l'aide d'une séquence peptidique d'au moins environ 10 à 15 acides aminés issue de la séquence délimitée par les acides aminés situés aux positions 206 et 338 de la séquence VIII.

L'invention a étalement pour objet l'utilisation des anticorps sus-mentionnés pour la mise en oeuvre de méthodes de diagnostic in vitro ainsi que dans des kits de diagnostic in vitro de l'infection d'un individu par C.pylori tels que décrits ci-dessus.

L'invention vise également des compositions immunogènes comprenant toute ou partie des polypeptides décrits ci-dessus, et plus particulièrement toute ou partie de la séquence polypeptidique délimitée par les acides aminés situés aux positions 206 et 338 de la séquence VIII, en association avec un véhicule pharmaceutiquement acceptable.

De telles compositions immunogènes sont utilisables en tant que compositions de vaccin pour la prévention de l'infection d'un individu par C.pylori.

L'invention concerne également des compositions pharmaceutiques comprenant un (ou plusieurs) anticorps tels que décrits ci-dessus, et plus particulièrement des anticorps susceptibles de former un complexe immunologique avec toute ou partie de la séquence peptidique délimitée par les aminés situés aux positions 206 et 338 de la séquence VIII, en association avec un véhicule pharmaceutiquement acceptable.

De telles compositions pharmaceutiques selon l'invention sont utilisables par le traitement des pathologies liées à l'infection d'un individu par C.pylori, notamment des gastrites, et des ulcères gastriques et duodénaux.

D'autres avantages et caractéristiques de l'invention sont rapportés dans la description qui suit concernant le clonage des gènes responsables de la production de la protéine à activité uréase chez C.pylori, le séquençage de la région associée à l'expression de la protéine à activité uréase et la spécificité de sondes nucléotidiques pour la détection de C.pylori.

Dans cette description, on se réfère aux figures 1 et 2 qui représentent :
- la figure 1, la carte de restriction d'un fragment PstI-EcoRI du cosmide recombinant IID2 portant l'information requise pour l'expression de l'uréase chez C.jejuni, et
- la figure 2, la carte de restriction d'un fragment d'environ 8kb de pILL590.

### 1) Réalisation d'une banque génomique dans un vecteur cosmidique navette; clonage des gènes impliqués dans la production de l'uréase chez C. pylori.

L'ADN chromosomique de la souche C.pylori (85P) est préparé de façon à générer des fragments d'ADN de haut poids moléculaire; environ 300»g de cet ADN chromosomique sont soumis à une digestion partielle controlée par l'endonucléase de restriction Sau3A et déposés sur gradient de sucrose de façon à isoler sélectivement des fragments de digestion compris entre 35 et 45kilobases. 1,5 ug de ces fragments sont ligaturés in vitro au vecteur navette-cosmide pILL575 linéarisé par l'endonuclése BamHI et déphosphorylé. pILL575 a été construit à partir du cosmide navette pILL550 décrit par Labigne-Roussel et al. dans J. Bacteriol. 169:5320-5323, 1987, dans lequel a été inséré le site "cos" du phage lambda (fragment Bgl II de 1,7 kb de pERG153 inséré au site Pvu II de pILL550); il est donc comme lui mobilisable par conjugaison, code pour une résistance à la kanamycine qui s'exprime à la fois chez E.coli et Campylobacter (Km^{r}) et se réplique à la fois chez E.coli et chez C.jejuni du fait de la présence de deux origines de réplication, l'une fonctionnelle chez E.coli exclusivement l'autre uniquement chez Campylobacter. Les produits de cette ligation sont empaquetés in vitro dans les particules de lambda puis introduits par transfection dans une souche de E.coli hébergeant un plasmide IncP capable de complémenter en trans les fonctions de transfert des cosmides recombinants. Les colonies de E.coli infectées et résistantes à la kanamycine sont immédiatement conservées individuellement à -80°C; une banque génomique cosmidique représentative de l'ensemble du génome de Campylobacter pylori est réalisée chez E.coli en conservant environ 500 clones indépendants.

Chacun des cosmides recombinants est alors transféré par conjugaison d'E.coli à une souche réceptrice de Campylobacter jejuni C31, naturellement urease⁻.

On vérifie pour chacun de ces transconjugants résistants à la kanamycine, s'il y a synthèse par Campylobacter jejuni d'une uréase.

On isole un cosmide sur 106 analysés portant une information génétique conférant à Campylobacteur jejuni C31 la capacité de produire une uréase. Le cosmide recombinant (IID2) a une taille de 54 kilobases. La position relative des sites de clivage par les endonucléases de restriction PstI, BamHI, SmaI et EcoRI sur cette séquence est présentée sur la figure 1.

### 2) Identification du ou des gènes requis pour la production d'uréase chez Campylobacter pylori.

On effectue le sous-clonage des gènes uréases en réalisant à partir de l'ADN cosmidique IID2 préparé chez E.coli des digestions partielles par l'endonucléase Sau3A de façon à générer des fragments de digestions partielles allant de 8 à 15 kilobases. On recherche le plus petit plasmide hybride cloné chez E.coli conférant l'aptitude à produire une uréase après son transfert par conjugaison à Campylobacter récepteur.

4 plasmides sur 37 testés portent l'information nécessaire à l'expression de l'uréase chez Campylobacter jejuni : pILL586, pILL587, pILL589 et pILL590 (cf.figure 2).

Par comparaison des cartes de restriction de ces quatre hybrides, on constate la présence d'une séquence de nucléotides de 4,2 kilobases commune aux quatre hybrides, suggérant qu'il s'agit là de la région requise pour l'expression de l'uréase. Un certain nombre de délétions ont été réalisées qui confirme ces conclusions (cf.figure 2).

Le fragment EcoRI-Pst I de pILL590 a été utilisé comme sonde dans des hybridations avec l'ADN total de C.pylori 85P en vue de s'assurer que les différents fragments de restrictions générés avec BamHI et HindIII à partir de pILL590 étaient présents dans 85P. Les résultats obtenus montrent que la séquence de 8 kb présente dans pILL590 n'a pas subi de réarrangements au cours des différentes étapes de clonage.

### 3) Séquence de nucléotides de la région associée à l'expression de l'uréase.

La séquence du fragment HindIII compris entre les sites H2 et H3 correspond à la séquence des 294 nucléotides de l'enchaînement (I).

La séquence suivante de 610 nucléotides de l'enchaînement (II) est interne au fragment défini par les sites H4 et B1 (Figure 2).

### 4) Spécificité des sondes nucléotidiques pour la détection de Campylobacter pylori.

Le fragment EcoRI-PstI de 8 kb provenant de pILL590 a été utilisé comme sonde radioactive dans des expériences d'hybridation.

32 Campylobacter pylori ont été testés en hybridation sur colonies et ont donné un signal positif; sur 35 Campylobacter jejuni, fétus et coli étudiés aucune n'a donné de signal positif dans des conditions d'hybridation stringentes classiques (50% de formamide, 37°, 3XSSC).

Dans ces mêmes conditions, la sonde C.pylori de 8 kilobases n'a pas donné de signal positif avec la liste des microorganismes suivants qui sont tous naturellement des producteurs d'uréase :
Klebsiella oxytoca (3 souches), Klebsiella pneumoniae (3 souches), Proteus mirabilis (3 souches) Proteus morganii (3 souches) Proteus rettgeri (3 souches) Proteus vulgaris (2 souches) Providencia stuartii (3 souches), Pseudomonas picketti (3 souches), Yersinia enterocolitica (3 souches), 5 souches d'Acinetobacter uréase ⁺, 2 souches d'Escherichia coli uréase ⁺ et 3 souches de Citrobacter freundii uréase ⁺.

L'absence de faux positif avec le fragment 8 kb garantit la spécificité des sondes nucléotidiques proposées.

Un procédé de préparation d'anticorps comprend:
- l'immunisation d'un animal à l'aide d'un polypeptide ci-dessus défini, et
- la récupération des anticorps formés selon les techniques classiques.

Un mode de préparation approprié des acides nucléiques (comportant au maximum 200 nucléotides - ou pb, lorsqu'il s'agit d'acides nucléiques bicaténaires) de l'invention par voie chimique comprend les étapes suivantes:
- la synthèse d'ADN en utilisant la méthode automatisée des β-cyanethyl phosphoramidite décrite dans Bioorganic Chemistry 4; 274-325, 1986,
- le clonage des ADN ainsi obtenus dans un vecteur plasmidien approprié et la récupération des ADN par hybridation avec une sonde appropriée.

Un mode de préparation, par vole chimique, d'acides nucléiques de longueur supérieure à 200 nucléotides - ou pb (lorsqu'il s'agit d'acides nucléiques bicaténaires) comprend les étapes suivantes:
- l'assemblage d'oligonucléotides synthétisés chimiquement, pourvus à leurs extrémités de sites de restriction différents, dont les séquences sont compatibles avec l'enchaînement en acides aminés du polypeptide naturel selon le principe décrit dans Proc. Nat. Acad. Sci. USA 80; 7461-7465, 1983,
- le clonage des ADN ainsi obtenus dans un vecteur plasmidien approprié et la récupération de l'acide nucléique recherché par hybridation avec une sonde appropriée.

## Revendications

1. Séquence de nucléotides caractérisée en ce qu'elle comprend dans sa structure une séquence codant pour une proteine à activité uréase telle qu'exprimée par C. pylori ou est capable de s'hybrider avec ladite séquence codante, laquelle est incluse dans un fragment EcoR1 (ClaI, BamH1) - PstI (HindIII) d'environ 8 kb dont la carte de restriction enzymatique est représentée sur la figure 2.

2. Séquence de nucléotides selon la revendication 1, caractérisée en ce qu'elle est incluse dans une partie d'un fragment d'environ 4,2 kb (± 5 %) délimité par les nucléotides d'extrémité situés respectivement à une distance d'environ 0,55 kb en amont du site H2 et d'environ 0,7 kb en aval du site B1 représentés sur la figure 2.

3. Séquence de nucléotides recombinante, caractérisée en ce qu'elle comprend une séquence selon l'une des revendications 1 ou 2, le cas échéant, associée avec un promoteur capable de contrôler la transcription de la séquence et une séquence d'ADN codant pour des signaux de terminaison de la transcription.

4. Séquence de nucléotides selon l'une des revendications 1 à 3, caractérisée en ce qu'elle est capable de s'hybrider avec une sonde nucléotidique formée à partir de la séquence présentant :
- l'enchaînement (I) de nucléotides suivant : 
AAGCTTTTTACCCGTGTTTACCCGTTTTATCAAAAATCTAAAA[?]CGCCCTTTCTTCTCAAGCA
ATTGTCCCTACAAACATGACCAATTTACGCCTTAAAGAATACTTAAAATCCCAAGATTTA
GAATTGAAGCATTCCGCGATTCCCCGATAAGTTTGTGACCGAATCCATCCGATTGAACAAA
GCCAATTTTCCGACCCGACCAAACGCCCCATATCATTTTTTACCGATTACGCTAAAACCGC
GATCCCTTCGTGTCCGCTTTCCAAGTGACCCGTTAGTGTTAGAAAGTAAGCTT
- ou l'enchaînement (IIbis) de nucléotides suivant : 
CCTAAAACGGATGACAACTATGTAAAAACAATTAAGGAGTAAGAAATGAAAAAGATTAGC
AGAAAAGAATATGTTTCTATGTATCGTCCTACTACACCCGATAAAGTGAGATTGCCGAT
CAGACTTGATCCCTGAAGTAGAACATGACTACACCATTTATCCGGAAGAGCTTAAATTCG
GTCCGCGTAAAACCCCTAAGAGAACCCATGACCCAATCTAACAACCCTAGCAAAGAAGAG
TTCGATTTAATTATCACTAACCCTTTAATCGTCGATTACACCGGTATTTATAAACGCGAT
ATTCGTATTAAAGATCCCAAAATCCCTCCCATTGGTAAACCCCGTAACAAAGACATCCAA
GATCCCGTTAAAAACAATCTTACCGTAGGTCCTCCTACTGAACCCTTAGCCCGGTGAAGGT
TTGATCGTAACCCCTGGTCGTATTGACACACACATCCACTTCATTTCACCCCAACAAATC
CCTACAGCTTTTCGAAGCGGTGTAACAACCATGATTCGTCGTCGAACCGGTCCTCCTGAT
GCCACTAATGCGACTACTATCACTCCAGCCAGAAGAAATTTAAAATGGATGCTCAGACGG
GCTGAAGAATATTCTATGAATTTAGGTTTCTTGCCTAAACGTAACCCTTCTAACGATCGG
AGCTTAGCCCGATCAAATTGAACCCCGTCCGATTGCCTTTAAAATTCACGAAGACTCGCGC
ACCACTCCTTCTGCAATCAATCATGCGTT
- ou l'enchaînement (V) de nucléotides suivant : 
1
ATGAAACTCAOOOCAAAAGAGTTAGATAAGTTGATGCTOCACTAOCCTGGAGAATTGGCT
AAAAAAOGCAAAGAAAAAGGCATTAAGCTTAACTATGTAGAAGCAGTAGCTTTGATTAGT
GOOCATATTATGGAAGAACOGAGAGCTGGTAAAAAGACTGOGGCTGAATTGATGCAAGAA
GGCOGCACTCTTTTAAAAOCAGATGATGTGATGGATGCOGTGGCAACCATGATOCATGAA
GTGGGTATTGAAGOGATGTTTOCTGATGGACTAAACTOGTAAOOGTGCATAOOOCTATT
GAGGOCAATGGTAAATTAGTTOCTGGTGAGTTGTTCTTAAAAATGAAGACATCACTATC
AAOGAAGGCAAAAAAGOOGTTAGOGTGAAAGTTAAAAATGTTGGOGACAGAOOGGTTCAA
ATOGGCTCACACTTOCATTTCTTTGAAGTGAATAGATGOCTAGACTTTGACAGAGAAAAA
ACTTTOGGTAAAOGCTTAGACATTGOGAGOGGGACACOGGTAAGATTTGAGOCTGGOGAA
GAAAAATOOGTAGAATTGATTGACATTGGOGGTAACAGAAGAATCTTTGGATTTAAOGCA
TTGGTTGATAGACAAGCAGACAAOGAAAGCAAAAAAATTGCTTTACACAGAGCTAAAGAG
OGTGGTTTTCATGGOGCTAAAAGOGATGACAACTATGTAAAAACAATTAAGGAGTAA
- ou l'enchaînement (VII) de nucléotides suivant : 
1
ATGAAAAAGATTACCAGAAAAGAATATGTTTCTATGTATGGTCCTACTACAGGCGATAAA
GTGAGATTGGCCGATACAGACTTGATCCCTGAAGTAGAACATGACTACACCATTTATGGC
GAAGAGCTTAAATTCGGTGGCGGTAAAACCCTAAGAGAACCCATGAGCCAATCTAACAAC
CCTACCAAAGAAGAGTTGGATTTAATTATCACTAACGCTTTAATCGTGGATTACACCGGT
ATTTATAAAGCGGATATTGGTATTAAAGATGGCAAAATCGCTGGCATTGGTAAAGCCGGT
AACAAAGACATGCAAGATGGCGTTAAAAACAATCTTAGCGTAGGTCCTGCTACTGAAGCC
TTAGCCGGTGAAGGTTTGATCGTAACGGCTGGTGGTATTGACACACACATCCACTTCATT
TCACCCCAACAAATCCCTACAGCTTTTGCAAGCGGTGTAACAACCATGATTGGTGGTGGA
ACCGGTCCTGCTGATGGCACTAATGCGACTACTATCACTCCAGGCAGAAGAAATTTAAAA
TGGATGCTCAGAGCGGCTGAAGAATATTCTATGAATTTAGGTTTCTTGGCTAAAGGTAAC
GCTTCTAACGATGCGAGCTTAGCCGATCAAATTGAAGCCGGTGCGATTGGCTTTAAAATT
CACGAAGACTGGGGCACCACTCCTTCTGCAATCAATCATGCGTTAGATGTTGCGGACAAA
TACGATGTGCAAGTCGCTATCCACACAGACACTTTGAATGAAGCCGGTTGTGTAGAAGAC
ACTATGGCTGCTATTGCTGGACGCACTATGCACACTTTCCACACTGAAGGCGCTGGCGGC
GGACACGCTCCTGATATTATTAAAGTAGCCGGTGAACACAACATTCTTCCCGCTTCCACT
AACCCCACCATCCCTTTCACCGTGAATACAGAAGCAGAGCACATGGACATGCTTATGGTG
TGCCACCACTTGGATAAAAGCATTAAAGAAGATGTTCAGTTCGCTGATTCAAGGATCCGC
CCTCAAACCATTGCGGCTGAAGACACTTTGCATGACATGGGGATTTTCTCAATCACCAGT
TCTGACTCTCAAGCGATGGGCCGTGTGGGTGAAGTTATCACTAGAACTTGGCAAACAGCT
GACAAAAACAAGAAAGAATTTGGCCGCTTGAAAGAAGAAAAAGGCGATAACGACAACTTC
AGGATCAAACGCTACTTGTCTAAATACACCATTAACCCAGCGATCGCTCATGGGATTAGC
GAGTATGTAGGTTCAGTAGAAGTGGGCAAAGTGGCTGACTTGGTATTGTGGAGTCCAGCA
TTCTTTGGCGTGAAACCCAACATGATCATCAAAGGCGGATTCATTGCGTTAAGCCAAATG
GGCGATGCGAACGCTTCTATCCCTACCCCACAACCGGTTTATTACAGAGAAATGTTCGCT
CATCATGGTAAAGCTAAATACGATGCAAACATCACTTTTGTGTCTCAAGCGGCTTATGAC
AAAGGCATTAAAGAAGAATTAGGACTTGAAAGACAAGTGTTGCCGGTAAAAAATTGCAGA
AATATCACTAAAAAAGACATGCAATTCAACGACACTACTGCTCACATTGAAGTCAATCCT
GAAACTTACCATGTGTTCGTGGATGGCAAAGAAGTAACTTCTAAACCAGCCAATAAAGTG
AGCTTGGCGCAACTCTTTAGCATTTTCTAG
1710
- ou les enchaînements de nucléotides complémentaires respectivement des enchaînements (I) (II) (V) et (VII) sus-mentionnés.

5. Séquence de nucléotides, caractérisée en ce qu'elle comprend un enchaînement de nucléotides selon la revendication 4 ou qu'elle est constituée par au moins une partie de cet enchaînement dès lors qu'elle est apte à reconnaître sans équivoque la présence d'une séquence selon l'une des revendications 1 à 3.

6. Séquence de nucléotides correspondant, selon le code génétique universel, à la séquence (III) en acides aminés suivante :

KLLGVLGVYQKSKNALSSQAIVATNMSNLALKEYLKSQDLELKHCAIGDKFVSEQMRLNK
ANFGGEQSGHIIFSDYAKTGDGLVCALQVSALVLESKL

7. Séquence de nucléotides correspondant, selon le code génétique universel, à la séquence (IV) en acides aminés suivante :

NMTTPFMAKSINSVAVKPLREGMSQSNNPSKEELDLIITNALIVDYTGIYKADIGIKDGK
IAGIGKGGNKDMQDGVKNNLSVGPATEALAGEGLIVTAGGIDTHIHFISPQQIPTAFRSG
VTTMIGGGTGPADGTNATTITPGRRNLKWMLRAAEEYSMNLGFLAKGNASNDASLADQIE
AGAIGFKIHEDWGTTPSAINHA

8. Séquence de nucléotides correspondant, selon le code génétique universel, à la séquence (VI) en acides aminés suivante :

1

MKLTPKELDKLMLHYAGELAKKRKEKGIKLNYVEAVALISAHIMEEARAGKKTAAELMQE

GRTLLKPDDVMDGVASMIHEVGIEAMFPDGTKLVTVHTPIEANGKLVPGELFLKNEDITI

NEGKKAVSVKVKNVGDRPVQIGSHFHFFEVNRCLDFDREKTFGKRLDIASGTAVRFEPGE

EKSVELIDIGGNRRIFGFNALVDRQADNESKKIALHRAKERGFHGAKSDDNYVKTIKE

239

9. Séquence de nucléotides correspondant, selon le code génétique universel, à la séquence (VIII) en acides aminés suivante :

1

MKKISRKEYVSMYGPTTGDKVRLGDTDLIAEVEHDYTIYGEELKFGGGKTLREGM

SQSNNPSKEELDLIITNALIVDYTGIYKADIGIKDGKIAGIGKGGNKDMQDGVKNN

LSVGPATEALAGEGLIVTAGGIDTHIHFISPQQIPTAFASGVTTMIGGGTGPAD

GTNATTITPGRRNLKWMLRAAEEYSMNLGFLAKGNASNDASLADQIEAGAIGF

KIHEDWGTTPSAINHALDVADKYDVQVAIHTDTLNEAGCVEDTMAAIAGRTMH

TFHTEGAGGGHAPDIIKVAGEHNILPASTNPTIPFTVNTEAEHMDMLMVCHHLD

KSIKEDVQFADSRIRPQTIAAEDTLHDMGIFSITSSDSQAMGRVGEVITRTWQT

ADKNKKEFGRLKEEKGDNDNFRIKRYLSKYTINPAIAHGISEYVGSVEVGKVADL

VLWSPAFFGVKPNMIIKGGFIALSQMGDANASIPTPQPVYYREMFAHHGKAKYD

ANITFVSQAAYDKGIKEELGLERQVLPVKNCRNITKKDMQFNDTTAHIEVNPETY

HVFVDGKEVTSKPANKVSLAQLFSIF_

569

10. Séquence de nucléotides, selon l'une quelconque des revendications 1 à 9, caractérisée en ce qu'elle porte l'information requise pour la production d'une protéine à activité uréase ou de fragments de cette dernière capables de former un complexe immunologique avec des anticorps respectivement contre la protéine à activité uréase telle qu'exprimée par C. pylori, ou contre des fragments de cette dernière.

11. Vecteur recombinant, notamment plasmide ou cosmide recombinant, capable de transformer une cellule hôte appropriée, ledit vecteur contenant une séquence de nucléotides selon l'une quelconque des revendications 1 à 10, le cas échéant sous le contrôle d'éléments de régulation permettant l'expression de ce fragment dans la cellule hôte.

12. Plasmide pILL590 porté par la souche E.coli S17-1 déposée le 16 août 1988 sous le numéro I-795 à la C.N.C.M.

13. Souche de micro-organisme transformée à l'aide d'au moins un vecteur selon la revendication 11 ou 12 ou d'une séquence de nucléotides selon l'une quelconque des revendications 1 à 10.

14. Protéine à activité uréase du type de celle exprimée chez C.pylori et ses fragments peptidiques, correspondant, selon le code génétique universel, aux séquences de nucléotides selon l'une quelconque des revendications 1 à 10.

15. Protéine à activité uréase du type de celle exprimée chez C.pylori, telle qu'obtenue par transformation de cellules hôtes au moyen d'un vecteur recombinant selon la revendication 11 ou 12, mise en culture, dans un milieu approprié, des cellules hôtes transformées et récupération de l'uréase à partir de ces cellules ou directement à partir du milieu de culture.

16. Anticorps polyclonal ou monoclonal, caractérisé en ce qu'il est dirigé contre tout ou partie de la protéine, ou de ses fragments, selon la revendication 14 ou 15.

17. Anticorps polyclonal ou monoclonal, caractérisé en ce qu'il est dirigé contre tout ou partie de la séquence déterminée par les acides aminés situés aux positions 206 et 338 de la séquence VIII selon la revendication 9.

18. Sonde de détection caractérisée en ce qu'elle comprend une séquence de nucléotides selon l'une quelconque des revendications 1 à 10.

19. Procédé de dépistage in vitro de la présence éventuelle de C. pylori dans un échantillon susceptible de le contenir, caractérisé en ce qu'il comprend les étapes suivantes :
- éventuellement l'amplification préalable d'une séquence selon l'une quelconque des revendications 1 à 12, susceptible d'être contenue dans l'échantillon, à l'aide d'amorces susceptibles respectivement de se lier, d'une part à l'extrémité 5' d'un brin de ladite séquence de nucléotides et d'autre part, à l'extrémité 3' de l'autre brin de ladite séquence de nucléotides.
- la mise en contact de l'échantillon biologique susmentionné avec une sonde nucléotidique selon la revendication 18, dans des conditions permettant la production d'un complexe d'hybridation formé entre ladite sonde et ladite séquence de nucléotides,
- la détection du susdit complexe d'hybridation.

20. Procédé de dépistage in vitro de la présence éventuelle de C. pylori dans un échantillon biologique susceptible de le contenir, caractérisé en ce qu'il comprend :
- la mise en contact de l'échantillon avec un anticorps selon la revendication 16 ou 17, dans des conditions permettant la production d'un complexe immunologique formé entre la protéine à activité uréase produite par C. pylori et cet anticorps, et
- la détection du complexe immunologique.

21. Nécessaire ou trousse pour la mise en oeuvre d'une méthode de dépistage in vitro de la présence éventuelle de C. pylori dans un échantillon, caractérisé en ce qu'il comprend :
- une quantité déterminée d'une sonde nucléotidique selon la revendication 18,
- avantageusement un milieu approprié à la formation d'une réaction d'hybridation entre la séquence à détecter, et la sonde sus-mentionnée,
- avantageusement des réactifs permettant la détection des complexes d'hybridation formés entre la séquence de nucléotides et la sonde lors de la réaction d'hybridation.

22. Nécessaire ou trousse pour la mise en oeuvre d'une méthode de dépistage in vitro de la présence éventuelle de C. pylori dans un échantillon, caractérisé en ce qu'il comprend :
- une quantité déterminée d'un anticorps selon la revendication 16 ou 17,
- avantageusement un milieu approprié à la formation d'une réaction immunologique entre au moins une partie d'une protéine à activité uréase produite par une souche de C. pylori et l'anticorps,
- avantageusement, des réactifs permettant la détection des complexes immunologiques formés entre au moins une partie de la protéine uréase et l'anticorps lors de la réaction immunologique.

23. Utilisation en tant qu'amorces dans des techniques d'amplification d'ADN, de type PCR ou autre, de deux séquences de nucléotides d'environ au moins 20 nucléotides, comprises dans l'une des séquences selon l'une quelconque des revendications 1 à 10 et distantes de 200 à 250 nucléotides environ, l'une des séquences étant capable de se lier à l'extrémité 5' d'un brin de la séquence à amplifier et la deuxième séquence à l'extrémité 3' de l'autre brin.

24. Composition pharmaceutique caractérisée en ce qu'elle comprend des anticorps selon la revendication 16 ou 17 en association avec un véhicule pharmaceutiquement acceptable.

25. Composition immunogène caractérisée en ce qu'elle comprend tout ou partie d'une protéine selon la revendication 14 ou la revendication 15 en association avec un véhicule pharmaceutiquement acceptable.

26. Composition selon la revendication 25 caractérisée en ce qu'elle comprend tout ou partie de la séquence délimitée par les acides aminés situés aux positions 206 et 338 de la séquence VIII selon la revendication 9.

## Claims

1. Sequence of nucleotides characterized in that it comprises in its structure a coding sequence for a protein with urease activity such as expressed by C.pylori, or in that it is capable of being hybridized with the said coding sequence, which is enclosed in a fragment EcoR1 (ClaI, BamHl) - PstI (HindIII) of approximately 8 kb and whose enzymatic restriction map is represented in Figure 2.

2. Sequence of nucleotides according to claim 1, characterized in that it is included in a part of a fragment of approximately 4.2 kb (± 5%) which is marked by the limiting nucleotides situated respectively at a distance of approximately 0.55 kb upstream of site H2 and approximately 0.7 kb downstream of site B1, as represented in Figure 2.

3. Recombinant nucleotides sequence, characterized in that it comprises a sequence according to one or other of claims 1 or 2, associated with a promoter which is capable of controlling the transcription of the sequence, and a DNA coding sequence for the termination signals of the transcription.

4. Sequence of nucleotides according to one of claims 1 to 3, characterized in that it is capable of being hybridized with a nucleotidic probe formed from the sequence having:
- the following chain (I) of nucleotides: 
AACCTTTTACGGGTGTTACGGGTTTATCAAAAATCTAAAACGCCTTTCTTCTCAAGCA
ATTGTCGCTACAAACATGACCAATTTACGCCTTAAAGAATACTTAAAATCCCAAGATTTA
GAATTGAAGCATTCGCGCATTCCCGATAAGTTTGTGACGCGAATCCATCCGATTGAACAAA
GCCAATTTTTCCACCCGACCAAACGCCCCCATATCATTTTTACCGATTACCCTAAAACCGC
GATCCCTTGGTGTCCGCTTTGCAAGTGACGCGGTTAGTGTTAGAAAGTAACCTT
- or the following chain (II bis) of nucleotides: 
CCTAAAACGGATGACAACTATGTAAAAACAATTAAGGAGTAAGAATGAAAAAGATTAGC
AGAAAAGAATATGTTTCTATGTATCGTCCTACTACACCCGATAAAGTGAGATTGCCGAT
CAGACTTGATCCCTGAAGTAGAACATGACTACACCATTTATCCGGAAGACCTTAAATTCG
GTCCCCGGTAAAACCCCTAAGAGAAGCCATGACCCAATCTAACAACCTAGCAAAGAAGAG
TTCGATTTAATTATCACTAACCCTTTAATCGTCGATTACACCGGTATTTATAAAGCGGAT
ATTCGTATTAAAGATCGCAAAATCCCTCCCATTGGTAAACCCGGTAACAAAGACATCCAA
GATCCCGTTAAAAACAATCTTACCGTACGTCCTGCTACTGAAGCCTTAGCCCGGTGAAGGT
TTGATCGTAACCCCTCGTCGTATTGACACACACATCCACTTCATTTCACCCCAACAAATC
CCTACAGCTTTTCGAAGCCGGTGTAACAACCATGATTCGTCGTCGAACCCGTCCTCCTGAT
CGCACTAATCCGACTACTATCACTCCAGCCAGAAGAAATTTAAAATCGATGCTCAGACCG
CCTGAAGAATATTCTATGAATTTACGTTTCTTGCCTAAAGGTAACCCTTCTAACGATCCG
ACCTTACCCGATCAAATTGAACCCCGGTCCGATTGCCTTTAAAATTCACGAAGACTCCCGC
ACCACTCCCTTCTGCAATCAATCATCCGTT
- or the following chain (V) of nucleotides: 
1
ATGAAACTCACCCCAAAAGAGTTAGATAAGTTGATGCTCCACTACGCTGGAGAATTGGCT
AAAAAACGCAAAGAAAAAGGCATTAAGCTTAACTATGTAGAAGCAGTAGCTTTGATTAGT
GCCCATATTATGGAAGAAGCGAGAGCTGGTAAAAAGACTGCGGCTGAATTGATGCAAGAA
GGGCGCACTCTTTTAAAACCAGATGATGTGATGGATGGCGTGGCAAGCATGATCCATGAA
GTCGGTATTGAAGCGATGTTTCCTGATGGGACTAAACTCGTAACCGTGCATACCCCTATT
GAGGCCAATGGTAAATTAGTTCCTGGTGAGTTGTTCTTAAAAAATGAAGACATCACTATC
AACGAAGGCAAAAAAGCCGTTAGCGTGAAAGTTAAAAATGTTGGCGACAGACCGGTTCAA
ATCGGCTCACACTTCCATTTCTTTGAAGTGAATAGATGCCTAGACTTTGACAGAGAAAAA
ACTTTCGGTAAACGCTTAGACATTCCGAGCGGGACACCGGTAAGATTTGAGCCTGGCGAA
GAAAAATCCGTAGAATTGATTGACATTGGCGGTAACAGAAGAATCTTTGGATTTAACGCA
TTGGTTGATAGACAAGCAGACAACGAAAGCAAAAAAATTGCTTTACACAGAGCTAAAGAG
CGTGGTTTTCATGGCGCTAAAAGCGATGACAACTATGTAAAAACAATTAAGGAGTAA
- or the following chain (VII) of nucleotides: 
1
ATGAAAAAGATTAGCAGAAAAGAATATGTTTCTATGTATCGTCCTACTACAGGCGATAAA
GTGAGATTCGCCGATACAGACTTGATCCCTGAAGTAGAACATGACTACACCATTTATGGC
GAAGACCTTAAATTCGGTCGCGGTAAAACCCTAAGAGAAGCCATGAGCCAATCTAACAAC
CCTAGCAAAGAAGAGTTCGATTTAATTATCACTAACGCTTTAATCGTGGATTACACCGGT
ATTTATAAAGCGGATATTGGTATTAAAGATGGCAAAATCGCTGGCATTGGTAAAGCCGGT
AACAAAGACATGCAAGATGGCGTTAAAAACAATCTTAGCGTAGGTCCTGCTACTGAAGCC
TTAGCCGGTGAAGGTTTGATCGTAACGGCTGGTGGTATTGACACACACATCCACTTCATT
TCACCCCAACAAATCCCTACAGCTTTTGCAAGCGGTGTAACAACCATGATTGGTGGTGGA
ACCGGTCCTGCTGATGGCACTAATGCGACTACTATCACTCCAGGCAGAAGAAATTTAAAA
TGGATGCTCAGAGCGGCTGAAGAATATTCTATGAATTTAGGTTTCTTGGCTAAAGGTAAC
GCTTCTAACGATGCGAGCTTAGCCGATCAAATTGAAGCCGGTGCGATTGGCTTTAAAATT
CACGAAGACTGGGGCACCACTCCTTCTGCAATCAATCATGCGTTAGATGTTGCGGACAAA
TACGATGTGCAAGTCGCTATCCACACAGACACTTTGAATGAAGCCGGTTGTGTAGAAGAC
ACTATGGCTGCTATTGCTGGACGCACTATGCACACTTTCCACACTGAAGGCGCTGGCGGC
GGACACGCTCCTGATATTATTAAAGTAGCCGGTGAACACAACATTCTTCCCGCTTCCACT
AACCCCACCATCCCTTTCACCGTGAATACAGAAGCAGAGCACATGGACATGCTTATGGTG
TGCCACCACTTGGATAAAAGCATTAAAGAAGATGTTCAGTTCGCTGATTCAAGGATCCGC
CCTCAAACCATTGCGGCTGAAGACACTTTGCATGACATGGGGATTTTCTCAATCACCAGT
TCTGACTCTCAAGCGATGGGCCGTGTGGGTGAAGTTATCACTAGAACTTGGCAAACAGCT
GACAAAAACAAGAAAGAATTTGGCCGCTTGAAAGAAGAAAAAGGCGATAACGACAACTTC
AGGATCAAACGCTACTTGTCTAAATACACCATTAACCCAGCGATCGCTCATGGGATTAGC
GAGTATGTAGGTTCAGTAGAAGTGGGCAAAGTGGCTGACTTGGTATTGTGGAGTCCAGCA
TTCTTTGGCGTGAAACCCAACATGATCATCAAAGGCGGATTCATTGCGTTAAGCCAAATG
GGCGATGCGAACGCTTCTATCCCTACCCCACAACCGGTTTATTACAGAGAAATGTTCGCT
CATCATGGTAAAGCTAAATACGATGCAAACATCACTTTTGTGTCTCAAGCGGCTTATGAC
AAAGGCATTAAAGAAGAATTAGGACTTGAAAGACAAGTGTTGCCGGTAAAAAATTGCAGA
AATATCACTAAAAAAGACATGCAATTCAACGACACTACTGCTCACATTGAAGTCAATCCT
GAAACTTACCATGTGTTCGTGGATGGCAAAGAAGTAACTTCTAAACCAGCCAATAAAGTG
AGCTTGGCGCAACTCTTTAGCATTTTCTAG
1710
- or the chains of nucleotides which are complementary respectively to the afore-mentioned chains (I) (II) (V) and (VII).

5. Sequence of nucleotides characterized in that it comprises a chain of nucleotides according to claim 4, or in that it is constituted by at least part of this chain when it is able to recognize unequivocally the presence of a sequence according to claims 1 to 3.

6. Sequence of nucleotides corresponding, according to the universal genetic code, to the following amino acids sequence (III):

KLLGVLGVYQKSKNALSSQAIVATNMSNLALKEYLKSQDLELKHCAIGDKFVSEQMRLNK
ANFGGEQSGHIIFSDYAKTGDGLVCALQVSALVLESKL

7. Sequence of nucleotides corresponding, according to the universal genetic code, to the following amino acids sequence (IV):

NMTTPFMAKSLNSVAVKPLREGMSQSNNPSKEELDLI ITNALIVDYTGIYKADIGIKDGK
IAGIGKGGNKDMQDGVKNNLSVGPATEALAGEGLIVTAGGIDTHIHFISPQQIPTAFRSG
VTTMIGGGTGPADGTNATTITPGRRNLKWMLRAAEEYSMNLGFLAKGNASNDASLADQIE
AGAIGFKIHEDWGTTPSAINHA

8. Sequence of nucleotides corresponding, according to the universal genetic code, to the following amino acids sequence (VI):

1
MKLTPKELDKLMLHYAGELAKKRKEKGIKLNYVEAVALISAHIMEEARAGKKTAAELMQE
GRTLLKPDDVMDGVASMIHEVGIEAMFPDGTKLVTVHTPIEANGKLVPGELFLKNEDITI
NEGKKAVSVKVKNVGDRPVQIGSHFHFFEVNRCLDFDREKTFGKRLDIASGTAVRFEPGE
EKSVELIDIGGNRRIFGFNALVDRQADNESKKIALHRAKERGFHGAKSDDNYVKTIKE
239

9. Sequence of nucleotides corresponding, according to the universal genetic code, to the following amino acids sequence (VIII):

1
MKKISRKEYVSMYGPTTGDKVRLGDTDLIAEVEHDYTIYGEELKFGGGKTLREGM
SQSNNPSKEELDLIITNALIVDYTGIYKADIGIKDGKIAGIGKGGNKDMQDGVKNN
LSVGPATEALAGEGLIVTAGGIDTHIHFISPQQIPTAFASGVTTMIGGGTGPAD
GTNATTITTPGRRNLKWMLRAAEEYSMNLGFLAKGNASNDASLADQIEAGAIGF
KIHEDWGTTPSAINHALDVADKYDVQVAIHTDTLNEAGCVEDTMAAIAGRTMH
TFHTEGAGGGHAPDIIKVAGEHNILPASTNPTIPFTVNTEAEHMDMLMVCHHLD
KSIKEDVQFADSRIRPQTIAAEDTLHDMGIFSITSSDSQAMGRVGEVITRTWQT
ADKNKKEFGRLKEEKGDNDNFRIKRYLSKYTINPAIAHGISEYVGSVEVGKVADL
VLWSPAFFGVKPNMIIKGGFIALSQMGDANASIPTPQPVYYREMFAHHGKAKYD
ANITFVSQAAYDKGIKEELGLERQVLPVKNCRNITKKDMQFNDTTAHIEVNPETY
HVFVDGKEVTSKPANKVSLAQLFSIF_
569

10. Sequence of nucleotides, according to any one of claims 1 to 9, characterized in that it carries the information required for the production of a protein with urease activity, or of fragments of this protein which are capable of forming an immunological complex with antibodies respectively against the protein with urease activity as expressed by C.pylori, or against fragments of this protein.

11. Recombinant vector, notably recombinant plasmid or cosmid, capable of transforming an appropriate host cell, the said vector containing a sequence of nucleotides according to any one of claims 1 to 10, possibly under the control of regulatory elements allowing the expression of the fragment in the host cell.

12. Plasmid pILL590 carried by strain E.coli S17-1 registered at the CNCM on 16th August 1988 under number I-795.

13. Micro-organism strain transformed by means of at least one vector according to claims 11 or 12 or by means of a sequence of nucleotides according to any one of claims 1 to 10.

14. Protein with urease activity, of the type expressed in C.pylori and its peptide fragments, corresponding, according to the universal genetic code, to the sequences of nucleotides according to any one of claims 1 to 10.

15. Protein with urease activity, of the type expressed in C.pylori, such as obtained through the transformation of host cells by means of a recombinant vector according to claims 11 or 12, culturing, in an appropriate environment, of the transformed host cells, and recovery of urease from these cells or directly from the culture medium.

16. Polyclonal or monoclonal antibody, characterized in that it is directed against all or part of the protein or its fragments, according to claims 14 or 15.

17. Polyclonal or monoclonal antibody, characterized in that it is directed against all or part of the sequence determined by the amino acids situated at positions 206 and 338 of sequence VIII, according to claim 9.

18. Detection probe characterized in that it comprises a sequence of nucleotides according to any one of claims 1 to 10.

19. Process for in vitro screening of the possible presence of C.pylori in a sample likely to contain the said C.pylori, characterized in that it comprises the following steps:
- possibly, the preliminary amplification of a sequence according to any one of claims 1 to 12, likely to be contained in the sample, by means of primers likely to be linked respectively, on the one hand, to 5' end of a strand of the said sequence of nucleotides and, on the other hand, to 3' end of the other strand of the said sequence of nucleotides,
- the coming into contact of the afore-mentioned biological sample with a probe according to claim 18, in conditions allowing the production of a hybridization complex formed between the said probe and the said sequence of nucleotides,
- the detection of the afore-mentioned hybridization complex.

20. Process for in vitro screening of the possible presence of C.pylori in a sample likely to contain the said C.pylori, characterized in that it comprises:
- the coming into contact of the sample with an antibody according to claims 16 or 17, in conditions allowing the production of an immunological complex formed between the protein with urease activity produced by C.pylori and this antibody, and
- the detection of the immunological complex.

21. Necessary material or kit for the implementation of a method for in vitro screening of the possible presence of C.pylori in a sample, characterized in that it comprises:
- a given quantity of a nucleotidic probe according to claim 18,
- advantageously, an appropriate medium for the formation of a hybridization reaction between the sequence to be detected and the afore-mentioned probe,
- advantageously, reagents allowing the detection of the hybridization complexes formed between the sequence of nucleotides and the probe during the hybridization reaction.

22. Necessary material or kit for the implementation of a method for in vitro screening of the possible presence of C.pylori in a sample, characterized in that it comprises:
- a given quantity of an antibody according to claims 16 or 17,
- advantageously, an appropriate environment for the formation of an immunological reaction between at least a part of a protein with urease activity produced by a strain of C.pylori, and the antibody.
- advantageously, reagents allowing the detection of the immunological complexes formed between at least a part of the urease protein and the antibody during the immunological reaction.

23. Use as primers in amplification techniques of DNA, such as PCR or other types, of two sequences of nucleotides of approximately at least 20 nucleotides, included in one of the sequences according to any one of claims 1 to 10 and approximately 200 to 250 nucleotides apart, one of the said two sequences being capable of attaching itself to 5' end of a strand of the sequence to be amplified, and the other sequence being capable of attaching itself to 3' end of the other strand.

24. Pharmaceutical composition characterized in that it comprises antibodies according to claims 16 or 17 in association with a pharmaceutically acceptable vehicle.

25. Immunogenic composition characterized in that it comprises all or part of a protein according to claims 14 or 15 in association with a pharmaceutically acceptable vehicle.

26. Composition according to claim 25 characterized in that it comprises all or part of the sequence marked by the amino acids situated in positions 206 and 338 of sequence VIII according to claim 9.

## Patentansprüche

1. Nucleotidsequenz, dadurch gekennzeichnet, daß sie in ihrer Struktur eine für ein Protein mit Ureaseaktivität, wie von C. pylori exprimiert, kodierende Sequenz enthält oder mit der genannten Kodierungssequenz hybridisieren kann, welche in einem Fragment Eco R1 (CLAI, BamH1) - PstI (HindIII) von etwa 8 kb enthalten ist, dessen enzymatische Restriktionskarte in Figur 2 wiedergegeben ist.

2. Nucleotidsequenz nach Anspruch 1, dadurch gekennzeichnet, daß sie in einem Teil eines Fragments von etwa 4,2 kb (± 5%), begrenzt von äußeren Nucleotiden enthalten ist, die jeweils in einer Entfernung von etwa 0,55 kb stromauf des Ortes H2 und von etwa 0,7 kb stromab des Ortes B1, wie in Figur 2 wiedergegeben, angeordnet sind.

3. Rekombinante Nucleotidsequenz, dadurch gekennzeichnet, daß sie eine Sequenz gemäß einem der Ansprüche 1 oder 2 enthält, gegebenenfalls assoziiert mit einem Promotor, der die Transkription der Sequenz kontrollieren kann, und mit einer DNA-Sequenz, die für Terminationssignale der Transkription kodiert.

4. Nucleotidsequenz gemäß einem der Ansprüche 1 bis 3, dadurch gekennzeichnet, daß sie mit einer Nucleotidsonde hybridisieren kann, welche ausgehend von einer Sequenz mit
- der folgenden Kette (I) von Nucleotiden 
AACCTTTTACCCGTGTTACCCGTTTATCAAAATCTAAAACGCCCTTTCTTCTCAACCA
ATTGTCCCTACAAACATCACCAATTTACCCCTTAAAGAATACTTAAAATCCCAAGATTTA
GAATTGAACCATTCCCCCATTCCCCATAAGTTTGTGACCGAATCCATCCGATTGAACAAA
GCCAATTTTCCGACCCGACCCAAACCCCCCATATCATTTTTACCGATTACCCTAAAACCCC
GATCCCTTCGGTGTCCCCTTTCCAAGTCACCCCGTTAGTGTTAGAAGTAACCTT
 oder der folgenden Kette (IIbis) von Nucleotiden 
CCTAAAACGGATGACAACTATGTAAAAACAATTAACGAGTAAGAATGAAAAGATTAGC
AGAAAAGAATATGTTTCTATGTATCGTCCTACTACACCGGATAAAGTGAGATTCCCGAT
CAGACTTGATCCCTGAAGTAGAACATGACTACACCATTTATCCGGAAGAGCTTAAATTCG
GTCCGCGTAAAACCCCTAAGAGAACCCATGACCCAATCTAACAACCCTAGCAAAGAAGAG
TTCGATTTAATTATCACTAACCCTTTAATCGTCGATTACACCGGTATTTATAAACGGAT
ATTCGTATTAAAGATCCCAAAATCCCTCCCATTCGTAAACCCCGTAACAAAGACATGCAA
GATCCCGTTAAAAACAATCTTACCGTACGTCCTGCTACTGACCCTTAGCCCGTGAAGGT
TTGATCGTAACCCCTCGTCGTATTGACACACACATCCACTTCATTTCACCCCAACAAATC
CCTACACCTTTTCGAAGCCGGTGTAACAACCATGATTCGTCGTCGAACCCGTCCTCCTGAT
CCCACTAATCCGACTACTATCACTCCACCCAGAAGAAATTTAAAATCGATGCTCAGACCG
CCTGAAGAATATTCTATGAATTTACGTTTCTTCCCTAAACGTAACCCTTCTAACCATCCG
ACCTTACCCCGATCAAATTGAACCCCGTCCGATTCCCTTTAAAATTCACGAAGACTCCCCC
ACCACTCCCTTCTCCAATCAATCATCCGTT
 oder der folgenden Kette (V) von Nucleotiden 
1
ATGAAACTCACCCCAAAAGAGTTAGATAAGTTGATCGCTCCACTACCCTGGAGAATTGCCT
AAAAAACGCAAAGAAAAAGGCATTAACGTTAACTATGTAGAGCAGTACCTTTGATTAGT
GCCCATATTATCGAAGAACGCAGACCTGGTAAAAAGACTCGCCCTGAATTCATGCAAGAA
GCCCGCACTCTTTTAAAACCAGATCATGTCATCGATCCGGTGCCAAGCATCATCCATGAA
GTCGGTATTGAACGCATGTTTCCTGATCCGACTAAACTCGTAACGGTCCATACCCCTATT
GACGCCCAATCGTAAATTAGTTCCTCGTGAGTTGTTCTTAAAAATGAAGACATCACTATC
AACGAAGCCAAAAAACGCCGTTACGCGTGAAGTTAAAAATGTTCCGACAGACCGGTTCAA
ATCGCCTCACACTTCCATTTCTTTGAGTCAATAGATCCCTAGACTTTGACAGAGAAAA
ACTTTCCGTAAACGCTTAGACATTCCGACGCCGACACCGGTAAGATTTGACGCCTCCCGAA
GAAAATCCGTAGAATTGATTGACATTCCGCGTAACAGAAGATCTTTCGATTTAACGCA
TTCGTTGATAGACAACCAGACAACGAACCAAAAAAATTCCTTACACAGACCTAAAGAG
CGTCGTTTTCATCCCGCCTAAAACGCATGACAACTATGTAAAACAATTAACGAGTAA
 oder der folgenden Kette (VII) von Nucleotiden 
1
ATGAAAAGATTAGCAGAAAGAATATGTTTCTATGTATCGTCCTACTACACCGATAAA
GTCAGATTGCCCCATACAGACTTGATCCCTGAAGTAGAACTGACTACACCATTTATCGC
GAAGACCTTAAATTCCGTCCCCGTAAAACCTAAGAGAACCCATGACCCAATCTAACAAC
CCTACCAAAGAAGAGTTTCCATTTAATTATCACTAACGCTTTAATCGTGGATTACACCGGT
ATTTATAAACGCGATATTGGTATTAAAGATCCCAAAATCCCTGGCATTGGTAAAGCGGT
AACAAAGACATCCAAGATCCGGTTAAAAACAATCTTAGCGTAGGTCCTGCTACTGAACGC
TTTACGCCGTGAAGGTTTGATCGTAACCCCTCGTCGTATTGACACACACATCCACTTCATT
TCACCCCAACAAATCCCTACAGCTTTTGCAACGCGTGTAACAACCATGATTCGTCGTCGA
ACCCGTCCTCCTGATCGCACTAATCCGACTACTATCACTCCAGCCAGAAGAAATTTAAAA
TCGATCCTCAGACGCGCTGAAGAATATTCTATGAATTTACGTTTCTTCCCTAAACGTAAC
GCTTCTAACGATCCGAGCTTACGCCGATCAAATTGAACGCCGTCCGATTCCCTTTAAAATT
CACGAAGACTCCCCCACCACTCCTTCTCCAATCAATCATCCGTTAGATGTTCCGCACAAA
TACGATGTCCAAGTCCCTATCCACACAGACACTTTGAATGAACGCCGTTGTGTAGAAGAC
ACTATCCCTCCTATTCCTCGACCCACTATCCACACTTTCCACACTGAACGCGCTCGCGCC
CGACACCCTCCTGATATTATTAAAGTACGCCGTGAACACAACATTCTTCCCGCTTCCACT
AACCCCACCATCCCTTTCACCGTGAATACAGAAGCAGACCACATCGACATGCTTATCGTG
TGCCACCACTTCGATAAAACCATTAAAGAAGATGTTCAGTTCGCTGATTCAAGGATCCGC
CCTCAAACCATTCGCGCTGAAGACACTTTGCATGACATCCCGATTTTCTCAATCACCAGT
TCTGACTCTCAACGCGATCCCCCGTGTCCGTGAAGTTATCACTAGAACTTGCCAAACAGCT
GACAAAAACAAGAAAGAATTTCCCCCCTTGAAAGAAGAAAAGGCGATAACGACAACTTC
ACGATCAAACCCTACTTGTCTAAATACACCATTAACCCACGCGATCCCTCATCCGATTAGC
GAGTATGTACGTTCAGTAGAAGTCCCCAAAGTCCCTGACTTCGTATTGTCCAGTCCAGCA
TTCTTTCCCGTGAAACCCAACATGATCATCAAACCGCCGATTCATTCGGTTAACGCAAATG
CCGCATCCGAACGCTTCTATCCCTACCCCACAACCCGTTTATTACAGAGAAATGTTCGCT
CATCATCGTAAACCTAAATACGATCCAAACATCACTTTTGTGTCTCAACGCGCTTATGAC
AAAGCCATTAAAGAAGAATTACGACTTGAAAGACAAGTGTTCCCCGTAAAAAATTCCAGA
AATATCACTAAAAAAGACATGCAATTCAACGACACTACTCCTCACATTGAAGTCAATCCT
GAAACTTACCCATGTGTTCGTCCATCCCAAAGAAGTAACTTCTAAACCACGCCAATAAAGTG
AGCTTCCCGCAACTCTTTACCATTTTCTAG
1710
 oder von Ketten komplementarer Nucleotide bzw. jeweils von den obengenannten Ketten (I), (II), (V) und (VII) gebildet ist.

5. Nucleotidsequenz, dadurch gekennzeichnet, daß sie eine Nucleotidkette gemäß Anspruch 4 enthält oder daß sie aus mindestens einem Teil dieser Kette besteht, soweit sie zur eindeutigen Erkennung der Anwesenheit einer Sequenz gemäß einem der Ansprüche 1 bis 3 befähigt ist.

6. Nucleotidsequenz entsprechend gemäß dem universalen genetischen Code der folgenden Sequenz (III) von Aminosäuren:

KLLGVLGVYQKSKNALSSQAIVATNMSNLALKEYLKSQDLELKHCAIGDKFVSEDMRLNK
ANFGGEQSGHIIFSDYAKTGDGLVCALQVSALVLESKL

7. Nucleotidsequenz entsprechend gemäß dem universalen genetischen Code der folgenden Sequenz (IV) von Aminosäuren:

N-MTTPFMAKSLNSVAVKPLPEGMSQSNNPSKEELDLI ITNALIVDYTGIYKADIGIKDGK
IAGIGKGGNKDMQDGVKNNLSVGPATEALAGEGLIVTAGGIDTHIHFI SPQQIPTAFRSG
VTTMI GGGTGPADGTNATT ITPGRRNLKWMLRAAEEYSMNLGFLAKGNASNDASLADQIE
AGAIGFKIHEDWGTTPSAINHA

8. Nucleotidsequenz entsprechend gemäß dem universalen genetischen Code der folgenden Sequenz (VI) von Aminosäuren:

1
MKLTPKELDKLMLHYAGELAKKRKEKGIKLNYVEAVALISAHIMEEARAGKKTAAELMQE
GRTLLKPDDVMDGVASMIHEVGIEAMFPDGTKLVTVHTPIEANGKLVPGELFLKNED ITI
NEGKKAVSVKVKNVGDRPVQI GSHFHFFEVNRCLDFDREKTFGKRLDIASGTAVRFEPGE
EKSVELID IGGNRRIFGFNALVDRQADNESKKIALHRAKERGFHGAKSDDNYVKTIKE
239

9. Nucleotidsequenz entsprechend gemäß dem universalen genetischen Code der folgenden Sequenz (VIII) von Aminosäuren:

1
MKKISRKEYVSMYGPTTGDKVRLGDTDLIAEVEHDYTIYGEELKFGGGKTLREGM
SQSNNPSKEELDLIITNALIVDYTGIYKADIGIKDGKIAGIGKGGNKDMQDGVKNN
LSVGPATEALAGEGLIVTAGGIDTHIHFISPQQIPTAFASGVTTMIGGGTGPAD
GTNATTITPGRRNLKWMLRAAEEYSMNLGFLAKGNASNDASLADQIEAGAIGF
KIHEDWGTTPSAINHALDVADKYDVQVAIHTDTLNEAGCVEDTMAAIAGRTMH
TFHTEGAGGGHAPDIIKVAGEHNILPASTNPTIPFTVNTEAEHMDMLMVCHHLD
KSIKEDVQFADSRIRPQTIAAEDTLHDMGIFSITSSDSQAMGRVGEVITRTWQT
ADKNKKEFGRLKEEKGDNDNFRIKRYLSKYTINPAIAHGISEYVGSVEVGKVADL
VLWSPAFFGVKPNMIIKGGFIALSQMGDANASIPTPQPVYYREMFAHHGKAKYD
ANITFVSQAAYDKGIKEELGLERQVLPVKNCRNITKKDMQFNDTTAHIEVNPETY
HVFVDGKEVTSKPANKVSLAQLFSIF_
569

10. Nucleotidsequenz gemäß einem jeden der Ansprüche 1 bis 9, dadurch gekennzeichnet, daß sie die Information trägt, welche für die Produktion eines Proteins mit Ureaseaktivität oder Fragmenten desselben, die einen immunologischen Komplex mit Antikörpern jeweils gegen das Protein mit Ureaseaktivität, wie es von C.pylori exprimiert wird, oder gegen Fragmente desselben bilden können, erforderlich ist.

11. Rekombinanter Vektor, insbesondere rekombinantes Plasmid oder Cosmid, der eine geeignete Wirtszelle transformieren kann und der eine Nucleotidsequenz gemäß einem jeden der Ansprüche 1 bis 10 enthält, gegebenenfalls unter der Kontrolle von Regulationselementen, die die Expression dieses Fragments in der Wirtszelle gestatten.

12. Plasmid pILL590, getragen vom Stamm E.coli S17-1, hinterlegt am 16. August 1988 unter der Nummer I-795 bei der C.N.C.M.

13. Mikroorganismenstamm, transformiert mit Hilfe mindestens eines Vektors gemäß den Ansprüchen 11 oder 12 oder mit einer Nucleotidsequenz gemäß einem jeden der Ansprüche 1 bis 10.

14. Protein mit Ureaseaktivität vom Typ des bei C.pylori exprimierten und seine peptidischen Fragmente, entsprechend gemäß dem universalen genetischen Code den Nucleotidsequenzen gemäß einem jeden der Ansprüche 1 bis 10.

15. Protein mit Ureaseaktivität vom Typ des bei C.pylori exprimierten, erhalten durch Transformation von Wirtszellen mittels eines kombinanten Vektors gemäß den Ansprüchen 11 oder 12, Kultivierung der transformierten Wirtszellen in einem geeigneten Medium und Gewinnung der Urease aus diesen Zellen oder direkt aus dem Kulturmedium.

16. Polyklonaler oder monoklonaler Antikörper, dadurch gekennzeichnet, daß er gegen das ganze oder einen Teil des Proteins oder seine Fragmente gemäß den Ansprüchen 14 oder 15 gerichtet ist.

17. Polyklonaler oder monoklonaler Antikörper, dadurch gekennzeichnet, daß er gegen die ganze oder einen Teil der von den in den Positionen 206 und 338 der Sequenz VII gemäß Anspruch 9 angeordneten Aminosäuren bestimmten Sequenz gerichtet ist.

18. Erkennungssonde, dadurch gekennzeichnet, daß sie eine Nucleotidsequenz gemäß einem jeden der Ansprüche 1 bis 10 umfaßt.

19. Verfahren zum Nachweis in vitro der Anwesenheit von C.pylori in einer dieses möglicherweise enthaltenden Probe, dadurch gekennzeichnet, daß es die folgenden Schritte umfaßt:
- gegebenenfalls vorherige Vermehrung einer Sequenz gemäß einem jeden der Ansprüche 1 bis 12, die sich möglicherweise in Probe befindet, mit Hilfe von Primern, die einerseits an das 5'-Ende und andererseits an das 3'-Ende des anderen Stranges der Nucleotidsequenz binden können;
- Zusammenbringen der obengenannten biologischen Probe mit einer Nucleotidsonde gemäß Anspruch 18 unter Bedingungen, die die Bildung eines zwischen der genannten Sonde und der genannten Nucleotidsequenz ausgebildeten Hybridisierungskomplexes gestatten;
- Erkennung des genannten Hybridisierungskomplexes.

20. Verfahren zum Nachweis in vitro der Anwesenheit von C.pylori in einer dieses möglicherweise enthaltenden biologischen Probe, dadurch gekennzeichnet, daß es folgendes umfaßt:
- Zusammenbringen der Probe mit einem Antikörper gemäß dem Anspruch 16 oder 17 unter Bedingungen, die die Bildung des immunologischen Komplexes, ausgebildet zwischen dem Protein mit Ureaseaktivität, gebildet von C.pylori, und diesem Antikörper, gestatten
- Erkennung des immunologischen Komplexes.

21. Necessaire oder Kit für die Durchführung eines Verfahrens zur Erkennung in vitro der möglichen Anwesenheit von C.pylori in einer Probe, dadurch gekennzeichnet, daß es folgendes umfaßt:
- eine vorbestimmte Menge einer Nucleotidsonde gemäß Anspruch 18,
- vorzugsweise ein geeignetes Medium für die Ausbildung einer Hybridisierungsreaktion zwischen der zu erkennenden Sequenz und der obengenannten Sonde,
- vorzugsweise Reaktanten, die die Erkennung der zwischen der Nucleotidsequenz und der Sonde ausgebildeten Hybridisierungskomplexe im Verlauf der Hybridisierungsreaktion gestatten.

22. Necessaire oder Kit für die Durchführung eines Verfahrens zur Erkennung in vitro der möglichen Anwesenheit von C.pylori in einer Probe, dadurch gekennzeichnet, daß es folgendes umfaßt:
- eine vorbestimmte Menge eines Antikörpers gemäß den Ansprüchen 16 oder 17,
- vorzugsweise ein Medium, das zur Ausbildung einer immunologischen Reaktion zwischen mindestens einem Teil eines Proteins mit Ureaseaktivität, gebildet durch einen Stamm von C.pylori, und dem Antikörper geeignet ist,
- vorzugsweise Reaktanten, die die Erkennung der zwischen mindestens einem Teil des Ureaseproteins und dem Antikörper im Verlauf der immunologischen Reaktion gebildeten immunologischen Komplexe gestatten.

23. Verwendung als Primer in DNA-Vermehrungstechniken vom PCR-Typ oder anderen von zwei Nucleotidsequenzen mit mindestens etwa 20 Nucleotiden, enthalten in einer der Sequenzen gemäß einem jeden der Ansprüche 1 bis 10 und angeordnet im Abstand von etwa 200 bis 250 Nucleotiden, wobei die eine der Sequenzen an das 5'-Ende einer Kette der zu vermehrenden Sequenz und die andere Sequenz an das 3'-Ende der anderen Kette binden kann.

24. Pharmazeutische Zusammensetzung, dadurch gekennzeichnet, daß sie Antikörper gemäß dem Anspruch 16 oder 17 zusammen mit einem pharmazeutisch verträglichen Träger umfaßt.

25. Immunogene Zusammensetzung, dadurch gekennzeichnet, daß sie das Ganze oder einen Teil eines Proteins gemäß Anspruch 14 oder gemäß Anspruch 15 zusammen mit einem pharmazeutisch verträglichen Träger umfaßt.

26. Zusammensetzung nach Anspruch 25, dadurch gekennzeichnet, daß sie die ganze oder einen Teil der von den an den Positionen 206 und 338 angeordneten Aminosäuren der Sequenz VII gemäß Anspruch 9 begrenzten Sequenz umfaßt.
